(19) **Europäisches Patentamt European Patent Office Office européen des brevets**

(11) **EP 4 609 850 A1**

(12) **EUROPEAN PATENT APPLICATION**
published in accordance with Art. 153(4) EPC

(43) Date of publication:
03.09.2025 Bulletin 2025/36

(21) Application number: 23882560.8

(22) Date of filing: 20.10.2023

(51) International Patent Classification (IPC):
*A61K 8/81* (2006.01)       *A61K 8/06* (2006.01)
*A61Q 1/00* (2006.01)

(52) Cooperative Patent Classification (CPC):
A61K 8/06; A61K 8/81; A61Q 1/00

(86) International application number:
PCT/JP2023/038016

(87) International publication number:
WO 2024/090355 (02.05.2024 Gazette 2024/18)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 27.10.2022 JP 2022172406

(71) Applicant: **Toagosei Co., Ltd.**
**Minato-ku**
**Tokyo 105-8419 (JP)**

(72) Inventor: **SHIBATA, Hiroyuki**
**Nagoya-shi, Aichi 455-0026 (JP)**

(74) Representative: **TBK**
**Bavariaring 4-6**
**80336 München (DE)**

(54) **AQUEOUS POLYMER EMULSION FOR COSMETIC, PRODUCTION METHOD THEREFOR, AND COSMETIC**

(57) An aqueous polymer emulsion for cosmetics contains resin particles. The resin particles include a structural unit derived from a monomer (A) (e.g. ethyl(meth)acrylate), a structural unit derived from a monomer (C) (e.g. unsaturated carboxylic acid), and an optional structural unit derived from a monomer (B) (methyl ester group-containing unsaturated monomer), the amount of the monomer (A) unit is 70-99.9 mass%, that of the monomer (B) unit is 0-10 mass%, and that of the monomer (C) unit is 0.1-10 mass%, with respect to the total amount of the monomer units in the resin particles; 0.1-10 parts by mass of a nonionic surfactant having an HLB of 10.0-17.0 is contained with respect to the total amount of the monomers forming the resin particles; and an alcohol level derived from an alkyl group in the monomers is 150 ppm or lower.

EP 4 609 850 A1

**Description**

Technical Field

[Cross-Reference to Related Applications]

The present application claims a priority from Japanese Patent Application No. 2022-172406 filed on October 27, 2022, the entirety of the disclosure of which is incorporated herein by reference.
The present disclosure relates to a polymer emulsion for a cosmetic, to a method for producing the same, and to a cosmetic.

Background Art

In cosmetics such as a makeup cosmetic composition, a manicure preparation, a skin-care cosmetic composition, and a hair cosmetic composition, a water-soluble polymer has been used as a coating film-forming agent, from environmental and hygienic aspects. However, in recent years, an aqueous polymer emulsion is widely used as a coating film-forming agent instead of a water-soluble polymer having poor moisture resistance and water resistance.
From the viewpoint of usability of cosmetics and for daily UV-protection purpose, there is demand for cosmetics exhibiting a persistent cosmetic effect. Particularly in a hot and humid summer season and while playing sports, there is demand for cosmetics that are more excellent in water resistance, oil resistance (anti-sebum performance), and persistency in cosmetic effects (hereinafter may also be referred to as "cosmetic persistency"). However, a coating film formed from a conventionally employed aqueous polymer emulsion readily causes phenomena such as braking or peeling while wet, resulting in insufficient water resistance. Also, the coating film becomes cakey or runs by sebum, sweat, an oily component of a differently applied cosmetic, or the like, resulting in coming-off of the cosmetic. Thus, oil resistance is also insufficient.
In order to overcome these drawbacks, there has been proposed, for example, an aqueous polymer emulsion which is formed from solid and elastomeric particles of at least partially cross-linked polyorganosiloxane, and coating film-formable particles (see Patent Document 1).

Prior Art Documents

Patent Documents

Patent Document 1: Japanese Patent Application Laid-Open (kokai) No. 2000-355532

Summary of the Invention

Problems to be Solved by the Invention

However, in the aqueous polymer emulsion of Patent Document 1, since polyorganosiloxane particles and coating film-formable particles are both present as particles in an aqueous system, sufficient intrinsic coating film-formability cannot be attained. Thus, oil resistance is still insufficient, although a comparatively suitable water resistance has been achieved.
Also, such a conventional aqueous polymer emulsion generally contains microamounts of unreacted monomers and volatile organic compounds such as a decomposition product generated during polymerization, evoking the following problems. When the user applies the cosmetic such as a cosmetic base, a foundation, or a cream to the skin, an odor intrinsic to an volatile organic compound must be masked with a perfume or the like, in order to prevent provision of the user with the mal-odor (hereinafter may also be referred simply as "odor"). In this case, difficulty is encountered in provision of unscented cosmetics. Also, use of such conventional cosmetics is limited, when they are uses as point-make products such as cheek, mascara, and eyeshadow.
In order to reduce the volatile organic compound level in the aqueous polymer emulsion, an attempt of reducing a mal-odor of the aqueous polymer emulsion has been made. Specifically, the aqueous polymer emulsion obtained through emulsion polymerization is heated or subjected to another treatment, to thereby volatilize such volatile organic compounds. However, a previous study by the present inventor revealed that alcohol was generated over time in the aqueous polymer emulsion, despite performance of the aforementioned heat treatment for odor reduction. From the viewpoint of achieving odor reduction and enhanced storage stability, there is desired development of an aqueous polymer emulsion having a low volatile organic compound level maintained over a long period of time.
The present disclosure has been conceived in view of the foregoing. Thus, a main object of the disclosure is to provide an aqueous polymer emulsion for cosmetics, which emulsion can maintain a low volatile organic compound level over a long period of time and can form a coating film having excellent water resistance and oil resistance. Another object is to provide a cosmetic composition containing the aqueous polymer emulsion.

Means for Solving the Problems

The present inventor has conducted extensive studies to solve the aforementioned problems, and has found that a particular structure of monomer units present on the surfaces of resin particles or in the vicinity of the surfaces (or both cases) is gradually hydrolyzed, to thereby generate a volatile organic compound over time in the emulsion. Accordingly, the present disclosure provides the following means.

[1] An aqueous polymer emulsion for cosmetics, the emulsion containing resin particles, wherein the resin particles include a structural unit derived from a monomer (A), a structural unit derived from a monomer (C), and an optional structural unit derived from a monomer (B), wherein

the monomer (A) is an alkyl (meth)acrylate having a C2 to C12 alkyl group;
the monomer (B) is an unsaturated monomer having a methyl ester group; and
the monomer (C) is at least one monomer selected from the group consisting of an unsaturated carboxylic acid and an unsaturated carboxylic anhydride; the amount of the structural unit derived from the monomer (A) is 70 to 99.9 mass%, the amount of the structural unit derived from the monomer (B) is 0 to 10 mass%, and the amount of the structural unit derived from the monomer (C) is 0.1 to 10 mass%, with respect to the total amount of the structural units derived from the monomers forming the resin particles as 100 mass%; and the emulsion contains a nonionic surfactant having an HLB of 10.0 to 17.0 in an amount of 0.1 to 10 parts by mass, with respect to the total amount of the monomers forming the resin particles as 100 parts by mass, and has an alcohol level of 150 ppm or lower, the alcohol being derived from an alkyl group included in the monomers forming the resin particles.

[2] The aqueous polymer emulsion for cosmetics as described in [1] above, wherein the polymer forming the resin particles has a glass transition temperature of -20°C to 20°C.
[3] The aqueous polymer emulsion for cosmetics as described in [1] or [2] above, wherein the monomer (B) is methacrylic acid.
[4] The aqueous polymer emulsion for cosmetics as described in any of [1] to [3] above, wherein the monomer (B) is methyl (meth) acrylate.
[5] A method for producing an aqueous polymer emulsion for cosmetics as recited in any of [1] to [4] above, the method including a step of producing a polymer emulsion by conducting emulsion polymerization, under such conditions that the amount of the structural unit derived from the aforementioned monomer (A) is 70 to 99.9 mass%, the amount of the structural unit derived from the aforementioned monomer (B) is 0 to 10 mass%, and the amount of the structural unit derived from the aforementioned monomer (C) is 0.1 to 10 mass%, with respect to the total amount of the monomers used in the polymerization as 100 mass%; and a nonionic surfactant having an HLB of 10.0 to 17.0 is present in an amount of 0.1 to 10 parts by mass, with respect to the total amount of the monomers used in the polymerization as 100 parts by mass; a step including adding a basic compound to the polymer emulsion, to thereby neutralize the emulsion to a pH of 7.0 to 10.0, and heating at 50 to 90°C; and a step of removing a volatile organic compound in the aqueous polymer emulsion by supplying pressurized steam to a processing container that can provide the polymer emulsion after being heated with a reduced pressure, while the polymer emulsion is maintained in the processing container at a pressure controlled to 12 kPa to 57 kPa and in an internal state of the processing container as a water boiling condition.
[6] A cosmetic containing an aqueous polymer emulsion for cosmetics as recited in any of [1] to [4] above.
Advantageous Effects of the Invention

According to the present disclosure, there can be provided an aqueous polymer emulsion for cosmetics which emulsion can maintain a low volatile organic compound level over a long period of time and can form a coating film having excellent water resistance and oil resistance. Also, the aqueous polymer emulsion for cosmetic of the present disclosure can provide a cosmetic composition with excellent water resistance, oil resistance, and low-odor property. Modes for Carrying Out the Invention
Hereinafter, the present disclosure will be described in detail. Notably, as used herein, the term "(meth)acrylic" encompasses acrylic or methacrylic or both, the term "(meth)acrylate" encompasses acrylate or methacrylate or both. The numerical range with an expression of "a to b" refers to a numerical range from a or greater and b or less. Unless otherwise specified, an expression, for example, "0 to 5 mass%" refers to a range of "0 mass% or more and 5 mass% or less".

<Aqueous polymer emulsion for cosmetics>

The aqueous polymer emulsion for cosmetics of the present disclosure is an emulsion in which resin particles are dispersed in a solvent mainly containing water. As used herein, the expression "a solvent mainly containing water" refers to a solvent containing water in an amount of 70 mass% or more (upper limit: 100 mass%). The solvent preferably contains water in an amount of 80 mass% or more, more preferably 90 mass% or more, still more preferably 95 mass% or more, yet more preferably 99 mass% or more. The optionally used solvent other than water may be an hydrophilic solvent such as acetone. Hereinafter, the term "aqueous polymer emulsion for cosmetics" or "aqueous polymer emulsion" may also be referred to simply as "emulsion".

(Monomers forming the resin particles)

The emulsion of the present disclosure contains resin particles. A monomer forming the resin particles (i.e., a structural unit of a polymer forming the resin particles) may be a monomer obtained from a typical fossil resource material such as petroleum, or a monomer obtained from a biomass resource that is environmental-friendly (e.g., in relation to the greenhouse effect) (hereinafter may also be referred to as a "biomass monomer"). The biomass monomer, which is used in order to suppress of dependency on fossil resource materials, is preferably used as a monomer forming the resin particles.

The biomass monomer is synthesized from a biomass material; i.e., a material originating from a renewable organic resource. The term biomass material typically refers to a material derived from a bio-material which can be reproduced in a sustainable manner essentially in the presence sunlight, water, and carbon dioxide (typically, a plant undergoing photosynthesis). Therefore, a material derived from a fossil resource exhausted through use after mining (i.e., a fossil resource-based material) is excluded from the concept of biomass material used in the specification. The biomass monomer content and the biomass-originating carbon content of a polymer formed through polymerization of a biomass monomer can be estimated through a $^{14}$C isotope content as measured on the basis of ASTM D6866.

From the viewpoint of environmental consciousness, the ratio of biomass-originating carbon contained in the resin particles is preferably higher. When a biomass monomer is used as a monomer forming the resin particles, the biomass-originating carbon content is, for example, 10% or higher, preferably 20% or higher, more preferably 30% or higher, still more preferably approximately 100%.

The resin particles include, as a structural unit derived from a monomer forming the resin particles, a structural unit derived from (A) an alkyl (meth)acrylate having a C2 to C12 alkyl group (hereinafter may also be referred to as a "monomer (A)"), a structural unit derived from (C) at least one monomer selected from the group consisting of an unsaturated carboxylic acid and an unsaturated carboxylic anhydride (hereinafter may also be referred to as a "monomer (C)"), and, optionally, a structural unit derived from (B) an unsaturated monomer having a methyl ester group (-C(=O)-O-CH$_3$) (hereinafter may also be referred to as a "monomer (B)").

Specific examples of the monomer (A) include n-propyl (meth)acrylate, i-propyl (meth)acrylate, n-butyl (meth) acrylate, i-butyl (meth)acrylate, s-butyl (meth)acrylate, t-butyl (meth)acrylate, 2-ethylhexyl (meth)acrylate, n-octyl (meth) acrylate, and lauryl (meth)acrylate. These monomers may be used singly or in combination of two or more species. Of these, the monomer (A) is preferably an alkyl (meth)acrylate having a C4 to C8 alkyl group from the viewpoint of achieving more suitable water resistance and oil resistance of the emulsion formed therefrom. From the viewpoint of achieving enhancement in water resistance and oil resistance of the emulsion at low cost, the monomer (A) is preferably at least one species of n-butyl (meth)acrylate and 2-ethylhexyl acrylate.

The monomer (B), having a methyl ester group, considerably undergoes hydrolysis, to thereby generate methanol derived from the methyl ester group. Examples of the monomer (B) include compounds represented by the following formula (I):

$$CH_2=CR^1\text{-}CO\text{-}O\text{-}(R^2\text{-}CO\text{-}O)_n\text{-}CH_3 \qquad \cdots \text{ (I)}$$

wherein R$^1$ represents a hydrogen atom or a methyl group; R$^2$ represents a C1 to C6 alkylene group; and n is an integer of 0 to 2.

Specific examples of the monomer (B) include methyl (meth)acrylate and methyloxycarbonylethyl (meth) acrylate. Of these, methyl (meth)acrylate is preferred, from the viewpoints of low price and sufficiently achieving an effect enhancing of water resistance and oil resistance of the emulsion. In particular, methyl methacrylate is preferred, since the amount of alcohol (specifically, methanol) generated over time is relatively small, and an emulsion that can form a coating film with suitable water resistance and oil resistance can be produced.

Specific examples of the monomer (C) include acrylic acid, methacrylic acid, crotonic acid, maleic acid, fumaric acid, itaconic acid, citraconic acid, cinnamic acid, and maleic anhydride. These monomers may be used singly or in combination of two or more species. From the viewpoint of enhancement of water resistance and oil resistance of the emulsion at low cost, the monomer (C) is preferably acrylic acid or methacrylic acid, with methacrylic acid being more preferred.

The resin particles include a structural unit derived from the monomer (A) (hereinafter may also be referred to as "monomer (A) unit") in an amount of 70 to 99.9 mass%, a structural unit derived from the monomer (C) (hereinafter may also be referred to as "monomer (C) unit") in an amount of 0.1 to 10 mass%, and a structural unit derived from the monomer (B) (hereinafter may also be referred to as "monomer (B) unit") in an amount of 0 to 10 mass%, with respect to the total amount of the structural units derived from the monomers forming the resin particles as 100 mass%.

The relationship between the amounts of the monomer (A) unit, the monomer (B) unit, and the monomer (C) unit with the relating properties are as follows. When the monomer (A) unit content is 70 mass% or more, water resistance and cosmetic persistency can be generally be enhanced, and when it is 99.9 mass% or less, oil resistance and cosmetic persistency can be generally enhanced. When the relative amount of the monomer (B) unit is 10 mass% or less, the alcohol (specifically, methanol) level in the emulsion can be generally maintained at a low level during storage for a long period of time. When the relative amount of the monomer (C) unit is 0.1 mass% or more, stability over time and close adhesion can be generally enhanced, whereas when it is 10 mass% or less, water resistance and oil resistance can be generally enhanced.

From the aforementioned aspects, the monomer (A) unit content of the resin particles is preferably 75 mass% or more, with respect to the total amount of the structural units derived from the monomers forming the resin particles, more preferably 80 mass% or more, still more preferably 85 mass% or more, yet more preferably 90 mass% or more. The upper limit of the monomer (A) unit content is preferably 99.5 mass% or less, with respect to the total amount of the structural units derived from the monomers forming the resin particles.

The monomer (B) unit content of the resin particles is preferably 0 to 7 mass%, with respect to the total amount of the structural units derived from the monomers forming the resin particles, more preferably 0 to 5 mass%, still more preferably 0 to 3 mass%.

The monomer (C) unit content of the resin particles is preferably 0.1 mass% or more, with respect to the total amount of the structural units derived from the monomers forming the resin particles, more preferably 0.5 mass% or more, still more preferably 1 mass% or more. The upper limit of the monomer (C) unit content is preferably 8 mass% or less, with respect to the total amount of the structural units derived from the monomers forming the resin particles, more preferably 6 mass% or less.

The resin particles may further include a structural unit derived from a monomer differing from the monomers (A), (B), and (C) (hereinafter may also be referred to as an "additional monomer"). Examples of the additional monomer include alkyl (meth)acrylate compounds having a C1 or C2 alkyl group such as methyl (meth)acrylate and ethyl (meth)acrylate; alkyl (meth)acrylate compounds having a C≥9 alkyl group such as lauryl (meth)acrylate, stearyl (meth)acrylate, cetyl-eicosyl (meth)acrylate, and behenyl (meth)acrylate; radical-polymerizable compounds having a cyclic structure such as styrene, vinyltoluene, divinyltoluene, α-methylstyrene, p-methylstyrene, chlorostyrene, vinyldibenzyl chloride, benzyl (meth)acrylate, cyclohexyl (meth)acrylate, and isobornyl (meth)acrylate; hydroxy group-containing vinyl compounds such as 2-hydroxyethyl (meth)acrylate, 2-hydroxypropyl (meth)acrylate, and 2-hydroxy-3-phenoxypropyl (meth)acrylate; amino group-containing vinyl compounds such as dimethylaminomethyl (meth)acrylate, diethylaminomethyl (meth)acrylate, 2-dimethylaminoethyl (meth)acrylate, 2-diethylaminoethyl (meth)acrylate, 2-(di-n-propylamino)ethyl (meth)acrylate, 2-dimethylaminopropyl (meth)acrylate, 2-diethylaminopropyl (meth)acrylate, 2-(di-n-propylamino)propyl (meth)acrylate, 3-dimethylaminopropyl (meth)acrylate, 3-diethylaminopropyl (meth)acrylate, and 3-(di-n-propylamino) propyl (meth)acrylate; amide group-containing vinyl compounds such as (meth)acrylamide, N,N-dimethyl(meth)acrylamide, and N,N-dimethylaminopropyl(meth)acrylamide; sulfonic acid group-containing vinyl compounds such as methallylsulfonic acid and vinylsulfonic acid; polyoxyalkylene group-containing vinyl compounds such as an alcohol (meth)acrylate ester having a polyoxyethylene group or a polyoxypropylene group or both; alkoxy group-containing vinyl compounds such as 2-methoxyethyl (meth)acrylate, 2-ethoxyethyl (meth)acrylate, 2-(n-propoxy)ethyl (meth)acrylate, 2-(n-butoxy)ethyl (meth)acrylate, 3-methoxypropyl (meth)acrylate, 3-ethoxypropyl (meth)acrylate, 2-(n-propoxy)propyl (meth)acrylate, and 2-(n-butoxy)propyl (meth)acrylate; cyano group-containing (meth)acrylate ester compounds such as cyanomethyl (meth)acrylate, 1-cyanoethyl (meth)acrylate, 2-cyanoethyl (meth)acrylate, 1-cyanopropyl (meth)acrylate, 2-cyanopropyl (meth)acrylate, 3-cyanopropyl (meth)acrylate (meth)acrylate, 4-cyanobutyl (meth)acrylate, 6-cyanohexyl (meth)acrylate, 2-ethyl-6-cyanohexyl (meth)acrylate, and 8-cyanooctyl (meth)acrylate; cyanated vinyl compounds such as acrylonitrile, methacrylonitrile, and ethacrylonitrile; and vinyl ether compounds such as vinyl methyl ether, vinyl ethyl ether, vinyl n-butyl ether, vinyl phenyl ether, and vinyl cyclohexyl ether. These monomers may be used singly or in combination of two or more species.

The relative amount of the structural units derived from the additional monomer in the resin particles is preferably 25 mass% or less, with respect to the total amount of the structural units derived from the monomers forming the resin particles, more preferably 20 mass% or less, still more preferably 10 mass% or less, yet more preferably 5 mass% or less, further more preferably 1 mass% or less.

(Glass transition temperature)

The glass transition temperature (Tg) of the polymer forming the resin particles is preferably -20 to 20°C. When the glass transition temperature Tg is -20°C or higher, an emulsion that can provide appropriate adhesion can be produced. In this case, the cosmetic composition obtained from the emulsion can provide suitable sensation in use. When the glass transition temperature Tg is 20°C or lower, suitable film formability can be generally secured. From such viewpoints, the glass transition temperature Tg of the polymer forming the resin particles is more preferably -15°C or higher, still more preferably -10°C or higher. The upper limit of the glass transition temperature Tg of the polymer forming the resin particles is more preferably 15°C or lower, still more preferably 10°C or lower.

Notably, the glass transition temperature Tg of the polymer is calculated by the following numerical expression (1):

$$1/Tg = (Wa/Tga) + (Wb/Tgb) + (Wc/Tgc) + \cdots \qquad (1)$$

wherein Tg represents a glass transition temperature (unit: K) of the polymer; Tga, Tgb, Tgc, etc. represent glass transition temperatures (unit: K) of homopolymers of monomers a, b, c, etc., respectively; and Wa, Wb, Wc, etc. represent mass fractions of monomers a, b, c, etc. in the copolymers, respectively.

(Volume average particle size)

The resin particles contained in the emulsion of the present disclosure preferably has a volume average particle size of 70 to 200 nm. The volume average particle size of the resin particles is an average size of particles weighted by volume and is defined by the following numerical expression (2):

Volume average particle size = $\Sigma$(V1×d1 + V2×d2 + ... +Vi×di + ... + Vn×dn)/$\Sigma$(V1 + V2 + $\cdots$ + Vi + ... + Vn) = $\Sigma$(Vi×di)/$\Sigma$(Vi) $\cdots$ (2)　　　　(2)

wherein V represents a volume of particle, n represents the number of particles, and d represents a particle size.

When the volume average particle size of the resin particles is 70 nm or greater, the viscosity of the emulsion sufficiently decreases, whereby high-concentration cosmetics can be produced. When the volume average particle size of the resin particles is 200 nm or smaller, water resistance and oil resistance can be further enhanced. From those viewpoints, the volume average particle size of the resin particles is preferably 75 nm or greater, more preferably 80 nm or greater, still more preferably 85 nm or greater, yet more preferably 90 nm or greater. The upper limit of the volume average particle size of the resin particles is preferably 150 nm or smaller.

In the present specification, the volume average particle size is measured by means of a particle size analyzer (UPA-EX150, product of MicrotracBEL Corp.). Specifically the particle sizes are determined through the following procedure. Firstly, set zero (background measurement) is conducted at 25°C in water as a dispersion medium. Next, an aqueous polymer emulsion for cosmetics is prepared so as to have a solid concentration of 10 mass%, and the emulsion is diluted with water. A small volume of the thus-obtained liquid is added dropwise to a sample cell of the aforementioned particle size analyzer. After achievement of a uniform state of the test liquid in the sample cell, sample loading is conducted, and an indicated loading index is monitored. The loading index is regulated to fall within a range of 1.0 to 2.0, and then RUN (start of measurement) is conducted. The number of runs is set to 3, and the values of volume average particle size are averaged. The measurement time is set to 60 seconds, and the refractive index of the sample particles is set to 1.59. Water is used as a dispersion medium, and the refractive index of the dispersion medium is 1.33. The range of measurement is 0.0008 $\mu$m to 6.5400 $\mu$m.

(Nonionic surfactant)

The emulsion of the present disclosure contains a nonionic surfactant having an HLB of 10.0 to 17.0 (hereinafter may also be referred to as a "particular nonionic surfactant") in an amount of 0.1 to 10 parts by mass, with respect to the total amount of the monomers forming the resin particles as 100 parts by mass. The term "nonionic surfactant" refers to a surfactant exhibiting surface activity without dissociating to ions in aqueous solution. The HLB (i.e., hydrophilic-lipophilic balance) represents a partial molecular weight of a moiety corresponding to hydrophilic groups in the entire molecular weight of the surfactant and is determined through a Griffin's equation in relation to a nonionic surfactant.

When the surfactant is formed of two or more nonionic surfactants (i.e., a mixed surfactant), the HLB is calculated as an arithmetic means of HLBs of respective nonionic surfactants on the basis of the proportions of the surfactants. Specifically, the HLB is calculated by the following numerical expression (3):

$$\text{HLB of mixed surfactant HLB} = \Sigma(\text{HLBx} \times \text{Wx})/\Sigma\text{Wx} \quad \cdots \quad (3)$$

wherein HLBx represents an HLB value of nonionic surfactant X, and Wx represents a mass (unit :g) of the nonionic surfactant X having HLBx.

From the viewpoint of sufficiently lowering the amount of alcohol generated in the emulsion through hydrolysis during storage for a long period of time, the HLB of the particular nonionic surfactant contained in the emulsion of the present disclosure is preferably 11.0 to 17.0, more preferably 12.0 to 17.0, still more preferably 12.0 to 16.5, yet more preferably 12.5 to 16.5.

In the emulsion of the present disclosure, the amount of the particular nonionic surfactant is 0.1 to 10 parts by mass, with respect to the total amount of the monomers forming the resin particles contained in the emulsion as 100 parts by mass. When the particular nonionic surfactant content is less than 0.1 parts by mass, generally, the amount of the particular nonionic surfactant is too small, and the amount of alcohol generated in the emulsion through hydrolysis during storage for a long period of time cannot sufficiently be reduced. When the particular nonionic surfactant content is in excess of 10 parts by mass, generally, the coating film formed by use of the emulsion has poor water resistance and oil resistance, and sensation in use of the cosmetic produced from the emulsion is insufficient. From those viewpoints, the amount of the particular nonionic surfactant contained in the emulsion of the present disclosure is preferably 0.5 parts by mass or more, with respect to the total amount of the monomers forming the resin particles as 100 parts by mass, more preferably 0.75 parts by mass or more, still more preferably 1.0 part by mass or more, yet more preferably 1.2 parts by mass or more, further more preferably 1.5 parts by mass or more. The upper limit of the amount of the particular nonionic surfactant is preferably 8.0 parts by mass or less, with respect to the total amount of the monomers forming the resin particles as 100 parts by mass, more preferably 7.0 parts by mass or less, still more preferably 6.0 parts by mass or less, yet more preferably 5.5 parts by mass or less.

(Alcohol level)

A previous study by the present inventor revealed that alcohol generated over time in the emulsion containing the resin particles through hydrolysis of alkyl groups of the monomers forming the resin particles occurring over time in the emulsion. In this regard, in the emulsion of the present disclosure, generation of alcohol attributed to alkyl group of the monomers forming the resin particles is sufficiently suppressed, whereby the alcohol level in the emulsion can be maintained at a low value after storage for a long period of time. Specifically, the alcohol level in the emulsion of the present disclosure is 150 ppm or lower, preferably 120 ppm or lower, more preferably 100 ppm or lower, still more preferably 75 ppm or lower, yet more preferably 50 ppm or lower, further more preferably 30 ppm or lower, yet further preferably 20 ppm or lower, further preferably 10 ppm or lower. The level is preferably a value approximately 0 ppm. In other words, in the emulsion of the present disclosure, the alcohol level is as low as 150 ppm or lower in the emulsion even after storage for a long period of time, achieving excellent storage stability.

Notably, as used herein, the expression "alcohol level is 150 ppm or lower in the emulsion even after storage for a long period of time" refers to that the alcohol level is 150 ppm or lower in the emulsion, after the emulsion is placed in a container tightly closed and allowed to stand at 50°C for 4 weeks.

Examples of the alcohol derived from an alkyl group of a monomer forming the resin particles include an alcohol derived from a $\geq$2C alkyl group included in the monomer (A), and an alcohol derived from a methyl moiety of a methyl ester group included in the monomer (B) (i.e., methanol). Examples of the alcohol generated from the monomers via hydrolysis include C1 to C4 alcohols. Specific examples include methanol, ethanol, n-propanol, i-propanol, n-butanol, i-butanol, s-butanol, and t-butanol. In particular, the monomer (B) readily undergoes hydrolysis to generate methanol. Also, since methanol is a cosmetic ingredient to be avoided, the methanol level of a cosmetic is desirably suppressed to a low level. According to the emulsion of the present disclosure, the alcohol (including methanol) level can be suppressed to a low level, and thus is suited for cosmetic uses.

(Emulsion production method)

The emulsion of the present disclosure can be produced through the following procedure. Specifically, a mixture containing the monomer (A), the monomer (C), and the monomer (B) serving as an optional component is subjected to emulsion polymerization, to thereby yield an aqueous polymer emulsion containing resin particles. The thus-obtained emulsion is neutralized and heated. Thereafter, a volatile organic compound is removed from the emulsion. More specifically, the emulsion of the present disclosure is preferably produced through a method including the following polymerization step, neutralization/heating step, and deodorization step.

(1) Polymerization step:

Conducting emulsion polymerization, under such conditions that the amount of the structural unit derived from the monomer (A) is 70 to 99.9 mass%, that of the structural unit derived from the monomer (B) is 0 to 10 mass%, and that of the structural unit derived from the monomer (C) is 0.1 to 10 mass%, with respect to the total amount of the monomers used in the polymerization as 100 mass%; and a nonionic surfactant having an HLB of 10.0 to 17.0 is present in an amount of 0.1 to 10 parts by mass, with respect to the total amount of the monomers used in the polymerization as 100 parts by mass, to thereby yield an emulsion.

(2) Neutralization/heating step

Adding a basic compound to the polymer emulsion obtained in the polymerization step, to thereby neutralize the emulsion to a pH of 7.0 to 10.0, and heating at 50 to 90°C.

(3) Deodorization step

Supplying pressurized steam to a processing container that can provide the polymer emulsion which has been heated in the neutralization/heating step with a reduced pressure, while the polymer emulsion is maintained in the processing container at a pressure controlled to 12 kPa to 57 kPa and in an internal state of the processing container as a water boiling condition, to thereby remove a volatile organic compound in the emulsion.
Hereinafter, the steps will be described in detail.

(1) Polymerization step

No particular limitation is imposed on the emulsion polymerization method, and the polymerization step may be conducted through, for example, a batch-format reaction method including feeding of the entirety of the monomers, surfactant(s), and water into a reaction container, and causing the mixture to be reacted; or a dropwise addition reaction method including gradually adding a monomer or monomers in a dropwise manner to be reacted into a reaction container during polymerization reaction. Of these, from the viewpoint of easily regulating heat generation during polymerization reaction, the dropwise addition reaction route is preferred. In order to further enhance polymerization stability, in a preferred mode of the dropwise addition reaction, a monomer or monomers, a surfactant, and water are mixed under stirring to thereby form a monomer pre-emulsion in an emulsion state, and the monomer pre-emulsion is gradually added dropwise to a reaction container during polymerization reaction.
The emulsion polymerization reaction is conducted in the presence of a nonionic surfactant (i.e., a particular nonionic surfactant) having an HLB of 10.0 to 17.0. When the amount of the particular nonionic surfactant employed in polymerization reaction is excessively small, the effect of reducing the amount of alcohol generated in the emulsion through hydrolysis during storage for a long period of time cannot be fully attained. Also, when the amount of the particular nonionic surfactant employed is in excess of 10 mass%, generally, water resistance and oil resistance of the provided emulsion, and sensation in use of cosmetic obtained from the emulsion are insufficient. Thus, the amount of the particular nonionic surfactant employed in polymerization reaction is preferably 0.5 parts by mass or more, with respect to the total amount of the monomers employed in polymerization as 100 parts by mass, more preferably 0.75 parts by mass or more, still more preferably 1.0 part by mass or more, yet more preferably 1.2 parts by mass or more, further more preferably 1.5 parts by mass or more. The upper limit of the amount of particular nonionic surfactant is preferably 8.0 parts by mass or less, with respect to the total amount of the monomers employed in polymerization as 100 parts by mass, more preferably 7.0 parts by mass or less, still more preferably 6.0 parts by mass or less, yet more preferably 5.5 parts by mass or less.
Notably, the amount of the particular nonionic surfactant contained in the finally yielded emulsion of the present disclosure is essentially 0.1 to 10 parts by mass, with respect to the total amount of the monomers forming the resin particles as 100 parts by mass, and the entire amount of the particular nonionic surfactant contained in the emulsion of the present disclosure is not necessarily derived from a particular nonionic surfactant used in the emulsion polymerization. That is, the particular nonionic surfactant may be fed to a reaction container before initiation of polymerization or after initiation of polymerization (i.e., during polymerization reaction), or to the emulsion after termination of polymerization. From the viewpoints of sufficiently reducing the amount of alcohol generated in the emulsion through hydrolysis during storage for a long period of time as well as productivity, in a preferred mode, at least a part of the particular nonionic surfactant to be used in production of the emulsion of the present disclosure is added to a reaction container before termination of polymerization. More preferably, the entirety of the particular nonionic surfactant is added to the reaction container before termination of polymerization.
The surfactant employed in the polymerization reaction may be a particular nonionic surfactant as a single component, or may further include a surfactant differing from particular nonionic surfactant (hereinafter may also be referred to as an "additional surfactant") in combination. Examples of the additional surfactant include a nonionic surfactant having an HLB less than 10.0 or in excess of 17.0, an anionic surfactant, a cationic surfactant, and a polymerizable surfactant. Of these, an anionic surfactant is preferred. As used herein, the term "anionic surfactant" refers to a surfactant that can form ions in aqueous solution, with a anion serving as a hydrophilic moiety. The term "cationic surfactant" refers to a surfactant that can form ions in aqueous solution, with a cation serving as a hydrophilic moiety. The term "polymerizable surfactant" refers to an anionic or nonionic surfactant having, in the molecule thereof, one or more unsaturated double bonds which can undergo radical polymerization.

Specific examples of the surfactant employed in emulsion polymerization for producing the emulsion of the present disclosure include the following. Examples of the nonionic surfactant include polyoxyethylene alkyl ethers such as polyoxyethylene lauryl ether, polyoxyethylene cetyl ether, polyoxyethylene stearyl ether, polyoxyethylene oleyl ether, and polyoxyethylene behenyl ether; polyoxyethylene alkylphenyl ethers such as polyoxyethylene octylphenyl ether and polyoxyethylene nonylphenyl ether; polyoxyethylene styrenated phenyl ether; polyoxyethylene distyrenated phenyl ether; sorbitan higher fatty acid esters such as sorbitan monolaurate, sorbitan monostearate, and sorbitan trioleate; polyoxyethylene sorbitan higher fatty acid esters such as polyoxyethylene sorbitan monolaurate and polyoxyethylene sorbitan monostearate; polyoxyethylene higher fatty acid esters such as polyoxyethylene monolaurate and polyoxyethylene monostearate; glycerin higher fatty acid esters such as monoglyceride oleate and monoglyceride stearate; polyoxyethylene polyoxypropylene alkyl ethers such as polyoxyethylene-polyoxypropylene block copolymer, polyoxyethylene-polyoxypropylene cetyl ether, and polyoxyethylene-polyoxypropylene decyltetradecyl ether; diethanolamides such as coconut acid diethanolamide, laurylic acid diethanolamide, myristic acid diethanolamide, palmitic acid diethanolamide, stearic acid diethanolamide, isostearic acid diethanolamide, and oleic acid diethanolamide; monoethanolamides such as coconut acid monoethanolamide, laurylic acid monoethanolamide, myristic acid monoethanolamide, palmitic acid monoethanolamide, stearic acid monoethanolamide, isostearic acid monoethanolamide, and oleic acid monoethanolamide; isopropanolamides such as coconut acid isopropanolamide, lauric acid isopropanolamide, myristic acid isopropanolamide, palmitic acid isopropanolamide, straric acid isopropanolamide, isostearic acid isopropanolamide, and oleic acid isopropanolamide; polyoxyethylene monoethanolamides such as polyoxyethylene coconut acid monoethanolamide, polyoxyethylene laurylic acid monoethanolamide, polyoxyethylene myristic acid monoethanolamide, polyoxyethylene palmitic acid monoethanolamide, polyoxyethylene stearic acid monoethanolamide, polyoxyethylene isostearic acid monoethanolamide, and polyoxyethylene oleic acid monoethanolamide; alkyl glucosides such as decyl glucoside and lauryl glucoside; alkyldimethylamine oxides such as lauryldimethylamine oxide and stearyldimethylamine oxide; maltitol hydroxyfatty acid alkyl ethers; alkylated polysacchrides; sucrose fatty acid esters; and fatty acid isopropanolamides. Notably, as a particular nonionic surfactant, a surfactant having an HLB of 10.0 to 17.0 appropriately selected from the above examples may be used.

Examples of the anionic surfactant include alkylarylsulfonate salts such as laurylbenzenesulfonate; polyoxyethylene alkyl ether sulfate salts such as polyoxyethylene lauryl ether sulfate; alkylsulfate salts such as laurylsulfate salts; polyoxyethylene polyoxypropylene alkyl ether sulfate salts; polyoxyethylene alkyl ether acetate salts such as polyoxyethylene lauryl ether acetate salt; alkylsulfosuccinate salts such as dialkylsulfosuccinate salt, polyoxyalkylene alkylsulfosuccinate salt, and dioctylsulfosuccinate salt; polyoxyethylene styrenated phenyl ether sulfate salts; polyoxyethylene distyrenated phenyl ether sulfate salts; higher fatty acid salts such as sodium oleate; polyoxyethylene fatty acid amide ether sulfate salts; coconut acid methyltaurate salts; N-acyl-L-asparetate salts; coconut acid ethyl ester sulfonate salts; alkyl-$\beta$-alanine salts; acylmethyltaurate salts; alkylethanesulfonate salts; polyoxyethylene alkyl ether carboxylate salts; alkanesulfonate salts; olefinsulfonate salts; alkylphosphate salts; polyoxyalkylene alkyl ether phosphate salts; alkyl(or alkenyl)phosphate salts; and alkylamide phosphate salts.

Examples of cationic surfactant include alkyl tirmethylammonium salts such as lauryl trimethylammonium chloride; dialkyldimethylammonium salts such as distearyldimethylammonium chloride; alkyldimethylethylammonium salts such as octyldimethylethylammonium chloride; trialkylbenzylammonium salts such as tributylbenzylammonium chloride; alkyldimethylbenzylammonium salts such as lauryldimethylbenzylammonium chloride; alkylamine salts such as stearylamine hydrochloride; alkyldimethylaminopropylamides such as stearyldimethylaminopropylamide; and alkylpyridinium salts such as laurylpyridinium chloride.

Examples of the polymerizable surfactant include propenylalkylsulfosuccinate ester salts, (meth)acrylic acid polyoxyethylene sulfate salts, (meth)acrylic acid polyoxyethylene phospahe salts (e.g., Eleminol RS-30, product of Sanyo Chemical Industries, Ltd.), polyoxyethylene alkylpropenylphenyl ether sulfate salts (e.g., Aqualon HS-10, product of DKS Co., Ltd.), allyloxymethylalkyloxypolyoxyethylene sulfate salts (e.g., Aqualon KH-10, product of DKS Co., Ltd.), allyloxymethylnonylphenoxyethylhydroxy polyoxyethylene sulfate salts (e.g., Adeka Reasoap SE-10, product of ADEKA Corp.), allyloxymethylalkoxyethylhydroxy polyoxyethylene sulfate salts (e.g., Adeka Reasoap SR-10 and SR-30), polyoxyalkylenealkenyl ether sulfate salts (e.g., Latemul PD-104 and PD-105, product of Kao Corporation), bis(polyoxyethylene polycyclic phenyl ether) methacrylated sulfate salts (e.g., Antox MS-60, product of Nippon Nyukazai Co., Ltd.), allyloxymethylalkoxyethylhydroxy polyoxyethylene (e.g., Adeka Reasoap ER-20, product of ADEKA Corp.), polyoxyethylene alkylpropenylphenyl ether (e.g., Aqualon RN-20, product of DKS Co., Ltd.), and allyloxymethylnonylphenoxyethylhydroxy polyoxyethylene (e.g., Adeka Reasoap NE-10, product of ADEKA Corp.).

No particular limitation is imposed on the method of adding the surfactant(s) during polymerization reaction. However, from the viewpoint of achieving a sufficiently reduced volume average particle size of the resin particles, preferably, at least a part of the surfactant(s) employed in the polymerization reaction is added to the reaction container before initiation of polymerization, and then polymerization reaction is performed. In that case, the surfactant added to the reaction container before initiation of polymerization may be a particular nonionic surfactant, an additional surfactant, or a mixture thereof. Also, the surfactant may be added simultaneously with addition of a monomer or monomers or at a timing different from addition of the monomer(s). In accordance with need, the surfactant may be dissolved in water or the like, and the product is added thereto. In the polymerization reaction, after addition of the surfactant in an initial stage so as to initiate polymerization, boosting of surfactant may be conducted. Boosting of the surfactant may be carried out once or more times, or through continuous dropwise addition.

No particular limitation is imposed on the amount of the surfactant used in the polymerization (in the case of two or more surfactants, the total amount of the surfactants), and the amount is preferably 0.1 to 20 parts by mass, with respect to the total amount (as 100 parts by mass) of a monomer or monomers used in polymerization. When an appropriate amount of surfactant(s) is used, mechanical stability of the resin particles is more enhanced. When an appropriate amount of polymerizable surfactant(s) is used, mechanical stability of the resin particles is further enhanced. When the amount of the surfactant used is 0.1 parts by mass or more, suitable emulsion stability can be secured. Also, when the amount of the surfactant used is 20 parts by mass or less, suitable water resistance can be secured. Both cases are preferred. From those viewpoints, the amount of surfactant(s) used, with respect to the total amount of monomer(s) used in polymerization, is more preferably 0.2 parts by mass, still more preferably 0.5 parts by mass, yet more preferably 1.0 part by mass or more. The upper limit of the amount of surfactant(s) used is more preferably 17 parts by mass or less, with respect to the total amount of monomer(s) used in polymerization, still more preferably 15 parts by mass or less, yet more preferably 12 parts by mass or less.

In emulsion polymerization, a radical polymerization initiator (hereinafter may also be referred to simply as a "polymerization initiator") is preferably used. As the polymerization initiator, a known oil-soluble polymerization initiator or a known water-soluble polymerization initiator may be used.

Examples of the oil-soluble polymerization initiator include organic peroxides such as benzoyl peroxide, tert-butyl oxybenzoate, tert-butyl hydroperoxide, tert-butyl peroxy-2-ethylhexanoate, tert-butyl peroxy-3,5,5-trimethylhexanoate, di(tert-butyl) peroxide, cumene hydroperoxide, and p-menthane hydroperoxide; and azobis compounds such as 2,2'-azobisisobutyronitrile, 2,2'-azobis-2,4-dimethylvaleronitrile, 2,2'-azobis(4-methoxy-2,4-dimethylvaleronitrile), and 1,1'-azobiscyclohexane-1-carbonitrile.

Examples of the water-soluble polymerization initiator include ammonium persulfate, sodium persulfate, potassium persulfate, hydrogen peroxide, and 2,2'-azobis(2-methylpropionamidine) dihydrochloride.

In emulsion polymerization, a reducing agent may be used in combination with the polymerization initiator. By use of a reducing agent in combination, polymerization reaction can be accelerated. Examples of such a reducing agent include reducing organic compounds such as ascorbic acid, erythorbic acid, tartaric acid, citric acid, glucose, and a metal salt of formaldehyde bissulfoxylate or the like; reducing inorganic compounds such as sodium sulfite, sodium bisulfite, sodium metabisulfite (SMBS), and sodium hyposulfite; ferrous chloride, Rongalite, and thiourea dioxide.

In emulsion polymerization, a water-soluble polymerization initiator is preferably used as the polymerization initiator. The polymerization initiator is preferably used in an amount of 0.05 to 5 parts by mass, with respect to 100 parts by mass of a monomer mixture used for forming resin particles. The reducing agent is preferably used in an amount of 0.01 to 2.5 parts by mass, with respect to 100 parts by mass of the monomer mixture.

In emulsion polymerization for yielding the emulsion of the present disclosure, a buffer, a chain-transfer agent, or the like may be used in accordance with needs. Examples of the buffer include sodium acetate, sodium citrate, and sodium bicarbonate. Examples of the chain-transfer agent include 2-mercaptoethanol, octyl mercaptan, tert-dodecyl mercaptan, lauryl mercaptan, stearyl mercaptan, 2-ethylhexyl mercaptoacetate, octyl mercaptoacetate, 2-ethylhexyl mercaptopropionate, and octyl mercaptopropionate.

After completion of polymerization, a subsequent neutralization/heating step may be immediately carried out. However, for reducing the level of unreacted monomer(s) or for other reasons, a treatment of maintaining the polymerization temperature or thereabouts (e.g., a polymerization temperature $\pm 10°C$) for a predetermined time (i.e., aging treatment) is preferably conducted. The time of the aging treatment may be appropriately set and is, for example, 30 minutes to 5 hours. After termination of the aging treatment, there may be appropriately conducted an additional treatment of maintaining the emulsion at a temperature lower than the polymerization temperature.

(2) Neutralization/heating step

Examples of the basic compound employed in the neutralization/heating step include ammonia; alkylamines such as trimethylamine, triethylamine, and butylamine; alcoholamines such as 2-dimethylaminoethanol, diethylaminoethanol, diethanolamine, triethanolamine, and aminomethylpropanol; ether amines such as morpholine; basic amino acids such as arginine; and metal hydroxides such as potassium hydroxide and sodium hydroxide, in the form of aqueous solution.

The amount of the basic compound used may be appropriately tuned so that the pH of the emulsion after addition of the basic compound thereto (i.e., the pH of the emulsion during heat treatment) falls within a range of 7.0 to 10.0. From the viewpoint of achieving a sufficiently reduced amount of alcohol generated in the emulsion via hydrolysis during storage for a long period of time, the pH of the emulsion after addition of the basic compound thereto is preferably 7.2 to 10.0, more preferably 7.5 to 10.0, still more preferably 7.7 to 10.0.

No particular limitation is imposed on the method of heating the emulsion to which the basic compound has been added. In one possible mode, heating may be performed by means of a heating jacket, or provision of a processing container in which the emulsion is placed with a heat-exchanger or the like disposed around the container. Heating of the emulsion to which the basic compound has been added may be conducted under appropriate stirring.

In the neutralization/heating step, the temperature of the emulsion is adjusted to fall within a range of 50 to 90°C. When the temperature of the emulsion to which the basic compound has been added is lower than 50°C during heating of the emulsion, the rate of hydrolysis of the monomers forming the resin particles is retarded, whereby hydrolysis may fail to be sufficiently completed. As a result, alcohol generates over time in the emulsion during storage of the emulsion for a long period of time, possibly impairing storage stability of the emulsion. In contrast, when the temperature of the emulsion exceeds 90°C, a large mass of polymer coating film is formed on the inner wall of the processing container in which the emulsion is present (in particular, at the gas-liquid phase interface), possibly impairing the quality of the product due to a drop in nonvolatile component concentration. From those viewpoints, the temperature of heating the emulsion to which the basic compound has been added is preferably 50 to 85°C, more preferably 50 to 80°C, still more preferably 55 to 80°C. The heat treatment may be conducted under an atmospheric pressure, alternatively, under a reduced pressure.

The time of heating the emulsion to which the basic compound has been added, which varies depending on the pH and temperature of the emulsion, the species of the basic compound, etc., is preferably within 10 hours, from the viewpoint of productivity. When the emulsion obtained in the aforementioned polymerization step is subjected to a heat treatment for 1 to 5 hours after addition of the basic compound thereto, the alcohol level of the emulsion after storage for a long period of time can be maintained at 150 ppm or lower.

In the emulsion obtained through emulsion polymerization, substantially, the entirety or a most part of the formed polymer is present as resin particles in the emulsion. In some cases, a part of the formed polymer is dissolved in the aqueous phase. A study of the present inventor previously revealed the following. Thai is, the monomer (A) unit forming the polymer present in the aqueous phase of the emulsion, and the monomer (B) unit optionally included in the polymer, and also the monomer (A) unit and the optionally present monomer (B) unit present on the surfaces of resin particles or in the vicinity of the surfaces (or both cases) gradually undergo hydrolysis during storage of the emulsion for a long period of time. As a result, an alcohol derived from an alkyl group in the monomer (A) unit and an alcohol derived from a methyl ester group in the optionally included monomer (B) unit are generated over time in the emulsion. In particular, among others, methyl (meth)acrylate readily undergoes hydrolysis, to thereby generate methanol.

Focusing on the aforementioned issues, according to the present disclosure, a particular nonionic surfactant is incorporated into the emulsion; a basic compound is added to the emulsion before the deodorization step, to thereby neutralize the emulsion; the emulsion is subjected to a heat treatment at a temperature falling within a specific range; and the deodorization treatment is conducted, instead of subjecting the emulsion produced through emulsion polymerization as is to the subsequent deodorization step, to thereby remove a volatile organic compound from the emulsion. Thus, the level of alcohol generated via hydrolysis during storage for a long period of time can be suppressed to a low level.

The present disclosure should not be limited by the reason why the level of alcohol generated via hydrolysis during storage for a long period of time can be suppressed to a low level through neutralizing a basic compound to the emulsion containing a particular nonionic surfactant, further heating the emulsion, and conducting the deodorization step. However, it is assumed that the interface between the aqueous phase and the surfaces of the particles is made unclear by the particular nonionic surfactant, whereby hydrolysis of the monomer (A) unit and the monomer (B) unit (an optional component) present in the vicinity of the surfaces of particles can also proceed. In other words, according to the present disclosure, the hydrolysis, which intrinsically proceeds gradually in the emulsion during storage for a long period of time, is intentionally evoked in the production of the emulsion. Thus, conceivably, generation of alcohol over time in the emulsion produced through the present production method could be suppressed during storage for a long period of time.

Notably, in the case where the aging treatment is conducted after completion of polymerization, the neutralization/heating step may be conducted through employment of the aging treatment. That is, in the alternative way, the emulsion is heated with a basic compound which has been added immediately after completion of polymerization, whereby aging and hydrolysis of the monomer (A) unit and the monomer (B) unit can be simultaneously performed. Yet alternatively, after neutralization of the emulsion containing the particular nonionic surfactant by adding a basic compound thereto and conducting further heating, an additional basic compound may be added to the emulsion for adjusting pH of the emulsion or other reasons.

(3) Deodorization step

Generally, in the emulsion produced through emulsion polymerization, a microamount of volatile organic compounds, including unpolymerized monomers and decomposition products generated in polymerization, are present. Also, in the emulsion after the aforementioned neutralization/heating step, an alcohol generated through adding a basic compound to the emulsion obtained in the polymerization step and heating (more specifically, an alcohol derived from an alkyl group included in a monomer forming the resin particles) is present as a volatile organic compound. When the volatile organic compound level in the emulsion is high, the volatile organic compound may undesirably cause mal-odor. Thus, preferably, the emulsion produced through the polymerization step and the neutralization/heating step is subjected to a treatment of removing volatile organic compounds present in the emulsion, to thereby reduce the odor level.

Examples of the technique of reducing the volatile organic compound level include a technique which includes heating of the emulsion to thereby volatilize volatile organic compounds remaining in the emulsion; a technique which includes causing gas (e.g., air) to pass through the gas phase on the emulsion; a technique which includes blowing steam into the emulsion; and a technique which includes distillating out volatile organic compounds under reduced pressure. From the viewpoint of sufficiently lowering the volatile organic compound level in the emulsion to thereby reduce mal-odor, preferably, pressurized steam is blown under reduced pressure to the emulsion after heating conducted in the aforementioned neutralization/heating step, to thereby reduce the volatile organic compound level in the emulsion to an extremely low level of 150 ppm or lower.

More specifically, in a preferred procedure, the emulsion produced through the aforementioned neutralization/-heating step is fed to a processing container that can provide reduced pressure. The pressure of the emulsion in the processing container is adjusted to 12 kPa to 57 kPa, and the state in the processing container is maintained in a water boiling state. Under such conditions, pressurized steam is supplied into the processing container. Also, steam and volatile organic compounds vaporized from the emulsion which stay in the gas phase of the processing container are removed to the outside of the system by means of a vacuum pump, to thereby remove the volatile organic compounds. As a result, an odor-reduced emulsion is yielded.

The temperature of the emulsion to which steam is blown is preferably adjusted to 50°C to 85°C. When the temperature of the emulsion to which steam is blown is adjusted to 50°C or higher, retardation in speed of removing the volatile organic compounds can be suppressed, whereby an odor-reduced emulsion can be efficiently produced. Also, when the temperature of the emulsion to which steam is blown is adjusted to 85°C or lower, formation of a large mass of polymer coating film on the inner wall of the processing container (in particular, at the gas-liquid phase interface) can be suppressed, whereby a drop in quality due to a decrease in non-volatile component concentration can be suppressed.

In order to maintain the water boiling state, the pressure in the processing container is preferably adjusted to fall within a range of 12 kPa to 57 kPa (a 90 mmHg to 430 mmHg as reduced to "mmHg") during blowing pressurized steam into the processing container. From the viewpoint of controlling the emulsion temperature to a lower temperature in the deodorization treatment and effectively suppressing generation of aggregates of polymer particles, the pressure in the container is preferably 12 kPa (90 mmHg) to 40 kPa (300 mmHg), more preferably 12 kPa (90 mmHg) to 30 kPa (225 mmHg).

In order to avoid bumping of the emulsion, the contents of the container is preferably stirred during blowing pressurized steam into the container. Since stirring facilitates provision of bubbles, a defoaming agent may be used in an appropriate amount to suppress bubbles. Examples of preferred defoaming agents include a polyether-based defoaming agent (e.g., SN Deformer PC, product of San Nopco Ltd.) and a silicone-based defoaming agent (e.g., KS-66, product of Shin-Etsu Chemical Co., Ltd.).

The employed pressurized steam preferably has a gage pressure of about 0.05 to about 0.50 MPa (temperature at 110 to 160°C), more preferably a gage pressure of 0.05 to 0.40 MPa, still more preferably a gage pressure of 0.10 to 0.30 MPa. The amount of pressurized steam fed to the processing container is preferably 5 to 100 parts by mass of pressurized steam, with respect to 100 parts by mass of the emulsion, more preferably 5 to 90 parts by mass, still more preferably 5 to 80 parts by mass. When the amount of pressurized steam to satisfy the aforementioned conditions, thermal hysteresis imposed on the emulsion can be suppressed, whereby loss of stability of the emulsion can be suppressed. In addition, the efficiency of removing volatile organic compounds can be fully enhanced.

The time required for steam treatment, which varies depending on the pressurized steam feeding rate and other conditions, is preferably 10 hours or shorter, from the viewpoint of productivity. In the emulsion of the present disclosure, the volatile organic compound level can be reduced to 150 ppm or lower through the steam treatment for 3 to 6 hours.

The non-volatile organic compound concentration of the emulsion of the present disclosure is preferably 30 to 70 mass%, more preferably 40 to 60 mass%. As used herein, the term "non-volatile organic compound concentration" refers to a mass of residue of a sample after heating at 155°C for 30 minutes in a hot air drier, or the ratio of the mass of the residue to the mass of the sample before heating.

The viscosity of the emulsion of the present disclosure, as determined by means of a B-type viscometer at 25°C and 12 rpm, is preferably 10 to 2,000 mPa·s, more preferably 10 to 500 mPa·s, still more preferably 10 to 300 mPa·s. The pH of the emulsion of the present disclosure is preferably 2 to 9.

<Composition of aqueous polymer emulsion for cosmetics>

By incorporating one or more of the below-described components and the like into the emulsion of the present disclosure, aqueous polymer emulsion compositions for cosmetics are produced. So long as the effects of the present disclosure are not impaired, ingredients generally incorporated into cosmetics may be incorporated to complete cosmetic formulations. The aqueous polymer emulsion composition for cosmetics may contain known ingredients such as a thickener, a film-formation aid, a plasticizer, a humectant, a UV-absorber, fatty acid soap, oil and fat, wax, hydrocarbon oil, ester oil, powder, a polymer compound, an antiseptic, an antioxidant, a pH-regulator, a chelating agent, and a coloring material.

Examples of the thickener include an alkali-swelling type thickener, an associable polyurethane, a carboxylvinyl polymer, a thickening polysaccahride, and a clay mineral.

Examples of the film-formation aid and the plasticizer include cellosolves such as methyl cellosolve, ethyl cellosolve, and butyl cellosolve; carbitols such as carbitol, dimethyl carbitol, diethyl carbitol, butyl carbitol, and dibutyl carbitol; carbonates such as ethylene carbonate, diethylene carbonate, and propylene carbonate; acetates such as cellosolve acetate, butyl cellosolve acetate, butyl carbitol acetate, and sucrose acetate; alcohols such as ethanol, propanol, butanol, pentanol, hexanol, benzyl alcohol, and 2-phenylethanol; diols such as ethylene glycol, propylene glycol, butylene glycol, and hexylene glycol; esters such as phthalic acid diester, adipic acid diester, succinic acid diester, sebacic acid diester, and abietic citric acid ester; benzoic acid esters such as sucrose benzoate; and diethylbenzene.

Examples of the humectant include sorbitol, xylitol, propylene glycol, dipropylene glycol, 1,3-butylene glycol, glycerin, diglycerin, polyethylene glycol, hyaluronic acid, chondroitin sulfate, a pyrrolidonecarboxylate salt, and a DL-pyrrolidonecarboxylate salt.

Examples of the UV-absorber include benzophenone derivatives such as 2-hydroxy-4-methoxybenzophenone, 2-hydroxy-4-methoxybenzophenone-5-sulfonic acid, sodium 2-hydroxy-4-methoxybenzophenone-5-sulfoate, dihydromethoxybenzophenone, sodium dihydroxymethoxybenzophenonesulfonate, 2,4-dihydroxybenzophenone, and tetrahydroxybenzophenone; benzoic acid derivatives such as p-aminobenzoic acid, ethyl p-aminobenzoate, glyceryl p-aminobenzoate, amyl p-dimethylaminobenzoate, and octyl p-dimethylaminobenzoate; methoxycinnamic acid derivatives such as ethyl p-methoxycinnamate, isopropyl p-methoxycinnamate, octyl p-methoxycinnamate, 2-ethoxyethyl p-methoxycinnamate, sodium p-methoxycinnamate, potassium p-methoxycinnamate, and glycerin p-methoxycinnamate mono-2-ethylhexanoate; salicylic acid derivatives such as octyl salicylate, phenyl salicylate, homomentyl salicylate, dipropylene glycol salicylate, ethylene glycol salicylate, myristyl salicylate, and methyl salicylate; urocanic acid; ethyl urocanate; ethyl urocanate ester; 4-tert-butyl-4'-methoxybenzoylmethane; 2-(2'-hydroxy-5'-methylphenyl)benzotriazole; 2-phenyl-5-methylbenzoxazole; methyl anthranylate, and 2-ethylhexyl dimethoxybenzylidenedioxoimidazolidinepropionate.

Examples of the fatty acid soap include C6 to 24 higher fatty acid alkali salts. The fatty acid may be saturated or unsaturated, and examples thereof include capric acid, lauric acid, myristic acid, palmitic acid, stearic acid, isostearic acid, arachidic acid, behenic acid, oleic acid, isooleic acid, linoleic acid, linolenic acid, and archidonic acid. No particular limitation is imposed on the alkali for neutralizing the fatty acid, so long as it is used for producing ordinary soap. Examples of the alkali include potassium hydroxide, sodium hydroxide, triethanolamine, N-methyltaurine, and ammonia.

Examples of fat and oil include avocado oil, camellia oil, evening primrose oil, turtle oil, macadamia nut oil, corn oil, mink oil, olive oil, rapeseed oil, egg yolk oil, sesame oil, apricot kernel oil, wheat germ oil, camellia oil, castor oil, linseed oil, safflower oil, cottonseed oil, perilla oil, soy bean oil, peanut oil, tea seed oil, Torreya nucifera oil, rice bran oil, Chinese tung oil, Japanese tung oil, Jojoba oil, germ oil, triglycerin, glycerin trioctanoate, glycerin triisopalmitate, cacao butter, coconut oil, hardened palm oil, palm oil, palm kernel oil, Japan wax kernel oil, hardened oil, hardened castor oil, and polyoxyethylene adducts thereof.

Examples of the wax include spermaceti, bees wax, high oxidation-value bees wax, shellac, mink wax, lanolin, lanolin acetate, liquid lanolin, carnauba wax, candelilla wax, rice bran wax, bran wax, tallow wax, cotton wax, bay berry wax, insect wax, montan wax, schefflera wax, jojoba wax, sugarcane wax, insect wax, isopropyl lanolin fatty acid, hexyl laurate, reduced lanolin, hard lanolin, shellac wax, polyoxyethylene lanolin alcohol ether, polyoxyethylene lanolin alcohol acetate, polyoxyethylene cholesterol ether, lanolin fatty acid polyethylene glycol, and polyoxyethylene hydrogenated lanolin alcohol ether.

Examples of the hydrocarbon oil include oil components such as paraffin, liquid paraffin, ozokerite, squalene, pristane, ceresin, Vaseline, and microcrystalline wax.

Examples of the ester oil include myristate esters such as isopropyl myristate, butyl myristate, myristyl myristate, isocetyl myristate, octyldodecyl myristate, and 2-hexyldecyl myristate; palmitate esters such as isopropyl palmitate, cetyl palmitate, isostearyl palmitate, 2-ethylhexyl palmitate, 2-hexyldecyl palmitate, and 2-heptylundecyl palmitate; stearate esters such as butyl stearate, isocetyl stearate, cholesteryl stearate, isocetyl isostearate, cholesteryl 12-hydroxystrarate, and N-alkylglycol isostearate; laurate esters such as isopropyl laurate and hexyl laurate; linoleate ester such as ethyl linoleate and isopropyl linoleate; octanoate esters such as cetyl octanoate, hexyldecyl dimethyloctanoate, octyldodecyl dimethyloctanoate, and cetyl isootanoate; oleate esters such as decyl oleate and oleyl oleate; sorbitan fatty acid ester oils such as sorbitan monolaurate, sorbitan monopalmitate, sorbitan monostearate, sorbitan sesquistearate, sorbitan tristearate, sorbitan monooleate, sorbitan sesquioleate, sorbitan trioleate, sorbitan monoisostearate, and sorbitan sesquiisostearate; polyoxyethylene sorbitan fatty acid esters such as polyoxyethylene sorbitan monococofatty acid ester, polyoxyethylene sorbitan monopalmitate, polyoxyethylene sorbitan monostearate, polyoxyethylene sorbitan monoisostearate, polyoxyethylene sorbitan tristearate, and polyoxyethylene sorbitan monooleate; polyoxyethylene sorbit fatty acid esters such as polyoxyethylene sorbit monolaurate, polyoxyethylene sorbit hexastearate, polyoxyethylene sorbit tetrastearate, and polyoxyethylene sorbit tetraoleate; lactate esters such as cetyl lactate and myristyl lactate; malate esters such as diisostearyl malate; adipate esters such as diisobutyl adipate, di-2-heptylundecyl adipate, and 2-hexyldecyl adipate; sebacate esters such as di-2-ethylhexyl sebacate and diisopropyl sebacate; succinate esters such as 2-ethylhexyl succinate; citrate esters such as triethyl citrate, triisocetyl citrate, triisoarachyl citrate, triisooctyl citrate, acetyl triethyl citrate, and acetyl tributyl citrate; polyhydric alcohol esters such as ethylene glycol di-2-ethylhexanoate, propylene glycol monocaprate, propylene glycol dicaprate, propylene glycol didecanate, glyceryl tri(2-ethylhexanoate), glyceryl tri(caprylate caprate), trimethylolpropane tri(2-ethylhexanoate), trimethylolpropane triisostearate, penaerythritol tetra(2-ethylhexanoate), glyceryl tri(2-ethylhexanoate), glyceryl trimyristate, decaglyceryl decastearate, decaglyceryl decaoleate, decaglyceryl decaisostearate, glyceryl di-2-heptylundecanate, polyoxyethylene glyceryl monostearate, polyethylene glycol glyceryl monooleate, diglyceryl monostearate, diglyceryl monoisostearate, diglyceryl monooleate, tetraglyceryl monostearate, polyglyceryl monooleate, polyglyceryl tristearate, polyglyceryl pentastearate, polyglyceryl pentaoleate, and neopentyl glycol dicaprate; lanolin acetate; dipenaerythritol fatty acid ester; tri-2-heptylundecanoic acid glyceride; castor oil fatty acid methyl ester; acetoglyceride; 2-octyldodecyl N-lauroyl-L-glutamate; and ethyl laurate.

No particular limitation is imposed on the powder, so long as it can be generally incorporated into a resin for use in cosmetics. Any of the powder may be used, not depending on the morphology of the powder (e.g., spherical, acicular, or plate-like), the particle size (e.g., fume, microparticle, or pigment-level), particle structure (e.g., porous or nonporous), etc.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, synthetic mica, mica, kaolin, sericite, muscovite, phlogopite, lepidolite, biotite, lepidolite, silicic acid, silicic anhydrate, aluminum silicate, magnesium silicate, aluminum magnesium silicate, calcium silicate, barium silicate, strontium silicate, metal tungstate salts, hydroxyapatite, vermiculite, Higilite, bentonite, montmorillonite, hectorite, zeolite, ceramic powder, calcium monohydrogenphosphate, alumina, aluminum hydroxide, boron nitride, boron nitride, and silica.

Examples of the organic powder include starch powder, polyamide powder, polyester powder, polyethylene powder, polypropylene powder, polystyrene powder, polyurethane powder, benzoguanamine powder, polymethylbenzoguanamine powder, tetrafluoroethylene powder, poly(methyl methacrylate) powder, cellulose, silk powder, nylon powder, nylon 12, nylon 6, styrene-acrylic acid copolymer, divinylbenzene-styrene copolymer, vinylic resin, urea resin, phenolic resin, fluororesin, silicon resin, acrylic resin, melamine resin, epoxy resin, polycarbonate resin, microcrystalline fiber powder, rice starch, and lauroyl lysine.

No particular limitation is imposed on the polymer compound, so long as it is generally blended with the resin for cosmetics. The polymer compound can be categorized into a natural polymer compound, a semi-synthesized polymer compound, and synthesized polymer compound.

Examples of the natural polymer compound include gum arabic, tragacanth gum, galactan, guar gum, carob gum, karaya gum, carrageenan, pectin, cannan, quince seed (marmelo), alkecoid (gasso extract), starch (rice, corn, potato, or wheat), glycyrrhizic acid, xanthan gum, dehydroxanthan gum, dextran, succinoglucan, pullulan, collagen, casein, albumin, and gelatin.

Examples of the semi-synthesized polymer compound include starch-based compounds such as carboxymethine starch, methylhydroxypropyl starch, modified potato starch, modified corn starch, and hydroxypropyl starch phosphate; cellulose-based polymers such as methylcellulose, nitrocellulose, ethylcellulose, hydroxypropylmethylcellulose, hydroxyethylcellulose, sodium cellulosesulfate, hydroxypropylcellulose, sodium carboxymethylcellulose (CMC), crystalline cellulose, and cellulose powder; and alginic acid-based polymer such as sodium alginate and propylene glycol alginate.

Examples of the synthesized polymer compound include vinylic polymers such as poly(vinyl alcohol), poly(vinyl methyl ether), polyvinylpyrrolidone, and a carboxyvinyl polymer (Carbopol); polyoxyethylene-based polymers such as polyethylene glycol 2000, 4000, and 6000; polyoxyethylene-polyoxypropylene copolymer-base polymer; acrylic polymers such as vinyl acetate-based polymer, poly(meth)acrylate ester, and polyacrylamide; polyethyleneimine; and cationic polymers. These polymer compound may assume any of liquid, microparticles, particles, etc. Also, the polymer compound may be converted to a dispersion or an emulsion.

Examples of the antiseptic include alkyl p-oxy benzoate, benzoic acid, sodium benzoate, sorbic acid, potassium sorbate, phenoxyethanol, and dehydroacetic acid, and salts thereof.

Examples of the antioxidant include tocopherol, butylhydroxyanisole, and dibutylhydroxytoluene.

Examples of the pH-adjuster include lactic acid, citric acid, glycolic acid, succinic acid, tartaric acid, dl-malic acid, potassium carbonate, sodium hydrogen carbonate, and ammonium hydrogen carbonate.

Examples of the chelating agent include alanine, sodium edetate, sodium polyphosphate, sodium metaphosphate, and phosphoric acid.

<Cosmetic>

The cosmetic of the present disclosure may be used as a makeup cosmetic, a skin cosmetic, a hair cosmetic composition, etc., by incorporating a suitably chosen additional ingredient into the aqueous polymer emulsion. Examples of the makeup cosmetic include eyeliner, eyeshadow, eyeblow, mascara, manicure, cheek rouge, foundation, and lipstick. Examples of the skin cosmetic include pack, sunscreen, milky lotion, cream, and skin lotion. Examples of the hair cosmetic include chemical hair dye, general hair dye, permanent liquid, hair bleach, hair foam, shampoo, and rinse.

Examples

The present disclosure will next be described in detail by way of examples, which should not be construed as limiting the disclosure thereto. Unless otherwise specified, "part(s)" and "%" denote part(s) by mass and mass%, respectively.

1. Production of aqueous polymer emulsions

[Example 1]

Production of aqueous polymer emulsion

<Emulsion polymerization step and neutralization/heating step>

Into a reaction container equipped with a stirrer, a thermometer, a condenser, a nitrogen-introduction pipe, and two dropping funnels, there were fed water (50 parts) and an anionic surfactant (Emal 2F-30; solution of sodium laurylsulfonate in water (solid content: 30%), product of Kao Corporation; hereinafter may also be referred to as "2F-30") (1.8 parts). The contents were heated to 80°C.

Subsequently, 2F-30 (5 parts), a nonionic surfactant (Emulgen 120; HLB 15.3; polyoxyethylene lauryl ether (solid content: 100%), product of Kao Corporation; hereinafter may also be referred to as "E-120") (3 parts), and water (50 parts) were added to a monomer mixture (100 parts) shown in Table 1 under mixing, to thereby prepare a monomer pre-emulsion.

The entire amount of the above-prepared monomer pre-emulsion and 5% aqueous ammonium persulfate (20 parts) serving as a polymerization initiator were continuously added dropwise to the reaction container through individual dropping funnels over 4 hours. Emulsion polymerization was conducted, while the liquid temperature was maintained at about 80°C. After completion of dropwise addition, the reaction mixture was maintained at 80°C for further 2 hours. Thereafter, the reaction mixture was cooled to 60°C, and the pH thereof was adjusted to 8.5 with aqueous ammonia. Subsequently, a heat treatment was conducted for 3 hours, and then the reaction mixture was cooled to 50°C. Then, a defoaming agent (SN Defoamer PC, product of San Nopco Ltd.) (0.1 parts) was added thereto, to thereby yield an aqueous polymer emulsion.

<Deodorization step (a step of blowing pressurized steam)>

A pressurized steam supply pipe and a gas exhaust pipe were attached to a flask in which the aqueous polymer emulsion was placed. The temperature of the liquid was elevated to 55°C, and then, pressurized steam (pressure: 0.2 MPa) was blown into the aqueous polymer emulsion (100 parts by mass) at a rate of 5 parts/Hr under stirring, while steam in the system was discharged by means of a vacuum pump through the gas exhaust pipe. As a result, the pressure in the flask was reduced to 15 kPa, and a water boiling state in the system was maintained. By blowing pressurized steam over 5 hours, 25 parts of steam was supplied in total. The pH of the thus-obtained emulsion was adjusted to 8 with aqueous ammonia, to thereby yield an odor-reduced aqueous polymer emulsion.

[Examples 2 to 15, and 21 to 32]

Production of aqueous polymer emulsions

The procedure of Example 1 was repeated, except that monomer compositions and the amount of the nonionic surfactant added to the reaction container and HLB thereof were modified as shown in Tables 1 and 2, to thereby yield aqueous polymer emulsions of Examples 2 to 15, and 21 to 32. Notably, the numerical data given in Tables 1 and 2 without unit refer to those with a unit of "part(s)", and a blank column denotes that no specific item was used.

[Example 16]

Production of aqueous polymer emulsion

The procedure of Example 9 was repeated, except that no deodorization step was conducted, to thereby yield an aqueous polymer emulsion of Example 16.

[Example 17]

Production of aqueous polymer emulsion

The procedure of Example 9 was repeated, except that the pressure in the container in the deodorization step was changed to 25 kPa (a water-non-boiling state), to thereby yield an aqueous polymer emulsion of Example 17.

[Examples 18 and 19]

Production of aqueous polymer emulsion

The procedure of Example 9 was repeated, except that the pH employed in the heat treatment was changed to a corresponding value shown in Table 2, to thereby yield aqueous polymer emulsions of Examples 18 and 19.

[Example 20]

Synthesis of aqueous polymer emulsion

The procedure of Example 9 was repeated, except that the temperature employed in the heat treatment was changed to a corresponding value shown in Table 2, to thereby yield an aqueous polymer emulsion of Example 20.

[Comparative Examples 1 to 5]

Production of aqueous polymer emulsion

The procedure of Example 1 was repeated, except that monomer compositions and the amount of the nonionic surfactant added to the reaction container and HLB thereof were modified as shown in Table 2, to thereby yield aqueous polymer emulsions of Comparative Examples 1 to 5.

[Comparative Example 6]

Production of aqueous polymer emulsion

The procedure of Example 9 was repeated, except that no heat treatment was conducted, to thereby yield an aqueous polymer emulsion of Comparative Example 6.

2. Evaluation

(1) Evaluation of aqueous polymer emulsions

The liquid properties and physical properties of the aqueous polymer emulsions of Examples 1 to 32 and Comparative Examples 1 to 6 were evaluated in the following manner.

<Non-volatile component concentration>

An aqueous polymer emulsion was heated at 155°C for 30 minutes by means of a hot air drier. The ratio of the residue was calculated as non-volatile component concentration of the aqueous polymer emulsion by the following

numerical expression:

$$\text{Non-volatile component concentration (\%)} = (W3/W2) \times 100$$

(wherein W2 represents a mass of emulsion (unit: g) before heating, and W3 represents a mass of residual component (unit: g) after heating).

<Viscosity>

Measured by means of a B-type viscometer at 25°C and 12 rpm.

<Volume average particle size of aqueous polymer emulsion>

The volume average particle size was measured by means of a particle size analyzer (UPA-EX150, product of MicrotracBEL Corp.).

<Glass transition temperature of polymer>

The glass transition temperature (Tg) of a polymer was calculated by the aforementioned numerical expression (1).

<Water resistance>

Water resistance of the aqueous polymer emulsion after drying was evaluated through the following procedure. Firstly, an aqueous polymer emulsion was applied onto a glass plate by means of an applicator so as to have a dry thickness of 50 $\mu$m, and air-dried at 23°C and 50%RH for 1 day, to thereby prepare a test piece. The thus-prepared test piece was immersed in distilled water for 1 day and rubbed with a water-absorbed sponge of a wear tester at a load of 100 g and 10 reciprocated rubbing motions. Occurrence of falling and peeling were evaluated on the basis of the following ratings.

◎: No change was observed in the coating film (good)
○: Slight peeling or falling was observed in the coating film (fair)
△: Peeling or falling was observed in about a half portion of the coating film (slightly bad)
X: Peeling or falling was observed in a considerable portion of the coating film (bad)

<Oil resistance>

A test piece prepared through the same method as employed in the aforementioned water resistance test was immersed in oleic acid for 1 day. The test piece was rubbed with an oleic acid-absorbed sponge of a wear tester at a load of 100 g and 10 reciprocated rubbing motions, and occurrence of falling and peeling were evaluated on the basis of the same ratings as employed in the aforementioned water resistance test.

<Softness>

An aqueous polymer emulsion was applied onto a polyethylene terephthalate film (thickness: 25 $\mu$m) so as to have a dry thickness of 25 $\mu$m and then, dried by means of a hot air drier at 50°C for 6 hours. The dried piece was air-dried at 23°C and 50%RH for 1 day, to thereby prepare a test piece formed of a polyethylene terephthalate film with a coating film formed thereon. The thus-obtained test piece was subjected to bending action 10 times, and cracking or falling of the coating film was evaluated on the basis of the following ratings.

O: No change was observed in the coating film (good)
△: Slight cracking or falling was observed in the coating film (slightly bad)
X: Peeling or falling was observed in the entire surface of the coating film (bad)

<Tackiness>

The surface of a test piece was traced with a finger, the test piece having been prepared through the same method as employed in the aforementioned softness test. The stickiness of the coating film (i.e., tackiness) was determined through sensation of touch and evaluated on the basis of the following ratings. The less the stickiness, the more suitable the sensation in use.

◎: No stickiness (good)
○: Slightly sticky (fair)
Δ: Sticky (slightly bad)
X: Very sticky (bad)

<Evaluation of odor originating from volatile organic compound>

An aqueous polymer emulsion (80 g) was placed in a glass container (100 mL) and closely sealed in the container with a cap. The container was maintained in a hot air drier at 40°C for 1 hour and the immediately removed from the drier. Opening the cap, the odor was evaluated through a sensory test, and the odor level was evaluated on the basis of the following ratings.

◎: Substantially no odor (very good)
○: Slight odor (good)
Δ: Odor (slightly bad)
X: Strong odor (bad)

<Volatile organic compound (unreacted monomer) level>

The volatile organic compound (i.e., an unreacted monomer) content was measured by means of a gas chromatograph (apparatus: GC-2014 (product of Shimadzu Corporation), column: DB-1 (product of GL Science), carrier gas: nitrogen, and detector: hydrogen flame ionization detector). In gas chromatographic analysis, an aqueous polymer emulsion (0.5 g), ethanol (4.0 g), and 20% aqueous calcium chloride (0.3 g) were mixed, and the mixture was allowed to stand for 10 minutes. The mixture was subjected to centrifugation at 12,000 rpm for 10 minutes, and the supernatant (1.5 g) was taken, to thereby provide an internal standard. A 1% ethylene glycol monomethyl ether acetate aqueous solution (0.05 g) was added thereto, to thereby provide an injection liquid sample of gas chromatography.

<Alcohol (derived from monomer (A)) level and methanol (derived from monomer (B)) level immediately after production>

The alcohol (derived from monomer (A)) level and the methanol (derived from monomer (B)) level of an aqueous polymer emulsion immediately after production were measured by means of a gas chromatograph (apparatus: GC-2014 (product of Shimadzu Corporation), column: DB-1 (product of GL Science), carrier gas: nitrogen, and detector: hydrogen flame ionization detector). In gas chromatographic analysis, an aqueous polymer emulsion (0.5 g), water (4.0 g), and 20% aqueous calcium chloride (0.3 g) were mixed, and the mixture was allowed to stand for 10 minutes, followed by conducting centrifugation at 12,000 rpm for 10 minutes. An aliquot (1.5 g) of the supernatant was taken, and a 1% ethylene glycol monomethyl ether acetate aqueous solution (0.05 g) serving as an internal standard was added thereto, to thereby provide an injection liquid sample of gas chromatography.

<Alcohol (derived from monomer (A)) level and methanol (derived from monomer (B)) level after storage for a long period of time>

Each of the aqueous polymer emulsions produced in the Examples and Comparative Examples was placed in a sealable container and maintained in a hot air drier at 50°C for 28 days. In a manner similar to determination of the alcohol (derived from monomer (A)) level and the methanol (derived from monomer (B)) level immediately after production, the alcohol (derived from monomer (A)) level and the methanol (derived from monomer (B)) level after storage for a long period of time were determined.

Each of the aqueous polymer emulsions of Examples 1 to 32 and Comparative Examples 1 to 6 were evaluated in terms of liquid property, water resistance, oil resistance, softness, tackiness, odor of volatile organic compound, volatile organic compound (unreacted monomer) content, alcohol (derived from monomer (A)) level and methanol (derived from monomer (B)) level immediately after production, and alcohol (derived from monomer (A)) level and methanol (derived from monomer (B)) level after storage for a long period of time. Tables 3 and 4 show the results.

[Table 1]

EP 4 609 850 A1

| | | | | Examples | | | | | | | | | | | | | | | | | |
|---|---|---|---|---|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 | 14 | 15 | 16 | 17 | 18 |
| Monomer/surfactant | Monomers | A | EMA | | | | 20 | | | | | | | | | | | | | | |
| | | | BMA | 62 | 70 | 58 | 37 | 62 | 44 | 33 | 59 | 55 | 51 | 60 | 67.8 | 67 | 66 | 55 | 55 | 55 | 55 |
| | | | BA | 35 | 10 | 24 | 40 | 35 | 40 | 44 | 35 | 37 | 37 | 34 | 32 | 32 | 31 | 37 | 37 | 37 | 37 |
| | | | 2EHA | | 17 | | | | | | | | | | | | | | | | |
| | | | LA | | | 15 | | | | | | | | | | | | | | | |
| | | B | MMA | | | | | | | | 3 | 5 | 9 | | | | | | 5 | 5 | 5 |
| | | | MA | | | | | | | | | | | 3 | | | | | | | |
| | | C | MAA | 3 | 3 | 3 | 3 | | 3 | 3 | 3 | 3 | 3 | 3 | 0.2 | 1 | 1.5 | 8 | 3 | 3 | 3 |
| | | | AA | | | | | 3 | | | | | | | | | 1.5 | | | | |
| | | Additional | St | | | | | | 13 | 17 | | | | | | | | | | | |
| | | | HEMA | | | | | | | 3 | | | | | | | | | | | |
| | | Subtotal | | 100 | 100 | 100 | 100 | 100 | 100 | 100 | 100 | 100 | 100 | 100 | 100 | 100 | 100 | 100 | 100 | 100 | 100 |
| | Nonionic surfactant | Type | | E-120 | E-120 | E-120 | E-120 | E-120 | E-120 | E-120 | E-120 | E-120 | E-120 | E-120 | E-120 | E-120 | E-120 | E-120 | E-120 | E-120 | E-120 |
| | | HLB | | 15.3 | 15.3 | 15.3 | 15.3 | 15.3 | 15.3 | 15.3 | 15.3 | 15.3 | 15.3 | 15.3 | 15.3 | 15.3 | 15.3 | 15.3 | 15.3 | 15.3 | 15.3 |
| | | Amount | | 3 | 3 | 3 | 3 | 3 | 3 | 3 | 3 | 3 | 3 | 3 | 3 | 3 | 3 | 3 | 3 | 3 | 3 |
| Tg [°C] of polymer | | | | -8.4 | -6.2 | -5.7 | -6.1 | -9.4 | -5.8 | -6.2 | -6.8 | -7.4 | -5.2 | -7.9 | -8.4 | -7.7 | -5.6 | -5.7 | -7.4 | -7.4 | -7.4 |
| Heat treatment | | Treatment | | Yes | Yes | Yes | Yes | Yes | Yes | Yes | Yes | Yes | Yes | Yes | Yes | Yes | Yes | Yes | Yes | Yes | Yes |
| | | Temp. [°C] | | 60 | 60 | 60 | 60 | 60 | 60 | 60 | 60 | 60 | 60 | 60 | 60 | 60 | 60 | 60 | 60 | 60 | 60 |
| | | Time [Hr] | | 3 | 3 | 3 | 3 | 3 | 3 | 3 | 3 | 3 | 3 | 3 | 3 | 3 | 3 | 3 | 3 | 3 | 3 |
| | | pH | | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 7.2 |
| Deodorization treatment | | | | Yes | Yes | Yes | Yes | Yes | Yes | Yes | Yes | No | Yes | Yes | Yes | Yes | Yes | Yes | No | Yes | Yes |
| Temp. [°C] in container during deodorization | | | | 55 | 55 | 55 | 55 | 55 | 55 | 55 | 55 | 55 | 55 | 55 | 55 | 55 | 55 | 55 | - | 55 | 55 |
| Pressure [kPa] in container during deodorization | | | | 15 | 15 | 15 | 15 | 15 | 15 | 15 | 15 | 15 | 15 | 15 | 15 | 15 | 15 | 15 | - | 25 | 15 |
| Time [Hr] of pressurized steam supply | | | | 5 | 5 | 5 | 5 | 5 | 5 | 5 | 5 | 5 | 5 | 5 | 5 | 5 | 5 | 5 | - | 5 | 5 |

19

[Table 2]

| | | Examples | | | | | | | | | | | | | | Comparative Exmples | | | | | |
|---|---|---|---|---|---|---|---|---|---|---|---|---|---|---|---|---|---|---|---|---|---|
| | | 19 | 20 | 21 | 22 | 23 | 24 | 25 | 26 | 27 | 28 | 29 | 30 | 31 | 32 | 1 | 2 | 3 | 4 | 5 | 6 |
| Monomers — A | EMA | | | | | | | | | | | | | | | | | | | | |
| | BMA | | | | | | | | | | | | | | | | | | | | |
| | BA | 55 | 51 | 55 | 55 | 55 | 55 | 55 | 62 | 62 | 62 | 62 | 62 | 54 | 70 | 31 | 46 | 51 | 51 | 51 | 55 |
| | 2EHA | 37 | 37 | 37 | 37 | 37 | 37 | 37 | 35 | 35 | 35 | 35 | 35 | 43 | 17 | 46 | 42 | 37 | 37 | 37 | 37 |
| | LA | | | | | | | | | | | | | | | | | | | | |
| B | MMA | 5 | 9 | 5 | 5 | 5 | 5 | 5 | | | | | | | | 20 | 12 | 9 | 9 | 9 | 5 |
| | MA | | | | | | | | | | | | | | | | | | | | |
| C | MAA | 3 | 3 | 3 | 3 | 3 | 3 | 3 | 3 | 3 | 3 | 3 | 3 | 3 | 3 | 3 | 3 | 3 | 3 | 3 | 3 |
| | AA | | | | | | | | | | | | | | | | | | | | |
| Additional | St | | | | | | | | | | | | | | | | | | | | |
| | HEMA | | | | | | | | | | | | | | 10 | | | | | | |
| | Subtotal | 100 | 100 | 100 | 100 | 100 | 100 | 100 | 100 | 100 | 100 | 100 | 100 | 100 | 100 | 100 | 100 | 100 | 100 | 100 | 100 |
| Nonionic surfactant | Type | E-120 | E-120 | E-120 | E-120 | E-120 | E-120 | E-120 | E-106 | E-108 | E-109P | E-123P | E106/E-123P | E-120 | E-120 | E-120 | E-120 | E-120 | E-103 | E-150 | E-120 |
| | HLB | 15.3 | 15.3 | 15.3 | 15.3 | 15.3 | 15.3 | 15.3 | 10.5 | 12.1 | 13.6 | 16.9 | 13.7 | 15.3 | 15.3 | 15.3 | 15.3 | 15.3 | 8.1 | 18.4 | 15.3 |
| | Amount | 3 | 3 | 0.5 | 1 | 1.5 | 7 | 9 | 3 | 3 | 3 | 3 | 1.5/1.5 | 3 | 3 | 3 | 3 | 14 | 3 | 3 | 3 |
| Tg [°C] of polymer | | -7.4 | -5.2 | -7.4 | -7.4 | -7.4 | -7.4 | -7.4 | -8.4 | -8.4 | -8.4 | -8.4 | -8.4 | -14.7 | 12.8 | -6.6 | -6.3 | -5.2 | -5.2 | -5.2 | -7.4 |
| Heat treatment | Treatment | Yes | Yes | Yes | Yes | Yes | Yes | Yes | Yes | Yes | Yes | Yes | Yes | Yes | Yes | Yes | Yes | Yes | Yes | Yes | No |
| | Temp. [°C] | 60 | 80 | 60 | 60 | 60 | 60 | 60 | 60 | 60 | 60 | 60 | 60 | 60 | 60 | 60 | 60 | 60 | 60 | 60 | - |
| | Time [Hr] | 3 | 3 | 3 | 3 | 3 | 3 | 3 | 3 | 3 | 3 | 3 | 3 | 3 | 3 | 3 | 3 | 3 | 3 | 3 | - |
| | pH | 9.8 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | 8.5 | - |
| Deodorization treatment | | Yes | Yes | Yes | Yes | Yes | Yes | Yes | Yes | Yes | Yes | Yes | Yes | Yes | Yes | Yes | Yes | Yes | Yes | Yes | Yes |
| Temp. [°C] in container during deodorization | | 55 | 55 | 55 | 55 | 55 | 55 | 55 | 55 | 55 | 55 | 55 | 55 | 55 | 55 | 55 | 55 | 55 | 55 | 55 | 55 |
| Pressure [kPa] in container during deodorization | | 15 | 15 | 15 | 15 | 15 | 15 | 15 | 15 | 15 | 15 | 15 | 15 | 15 | 15 | 15 | 15 | 15 | 15 | 15 | 15 |
| Time [Hr] of pressurized steam supply | | 5 | 5 | 5 | 5 | 5 | 5 | 5 | 5 | 5 | 5 | 5 | 5 | 5 | 5 | 5 | 5 | 5 | 5 | 5 | 5 |

[Table 3]

[Table 3]

EP 4 609 850 A1

| | | | Examples | | | | | | | | | | | | | | | | | |
|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 16 | 17 | 18 |
| Evaluation | Liq. Properties | Non-volatile component concentration [%] | 46.1 | 46.0 | 46.0 | 45.9 | 45.7 | 46.0 | 46.1 | 46.0 | 45.8 | 45.9 | 45.8 | 46.0 | 45.9 | 45.7 | 45.8 | 45.9 | 46.0 | 45.9 |
| | | Viscosity [mPa.s] | 50 | 60 | 55 | 60 | 55 | 60 | 50 | 55 | 55 | 55 | 55 | 55 | 60 | 70 | 100 | 55 | 60 | 55 |
| | | pH | 8.0 | 8.0 | 7.9 | 7.9 | 8.1 | 8.0 | 7.9 | 8.0 | 7.9 | 8.0 | 8.0 | 7.9 | 7.8 | 8.0 | 8.1 | 8.5 | 8.3 | 8.0 |
| | | Volume av. particle size | 112 | 117 | 114 | 116 | 114 | 115 | 118 | 114 | 116 | 112 | 117 | 118 | 116 | 115 | 112 | 114 | 117 | 116 |
| | Phys properties | Water resistance | ◎ | ◎ | ◎ | ◎ | ◎ | ◎ | ○ | ◎ | ◎ | ◎ | ○ | ◎ | ◎ | ○ | ○ | ◎ | ◎ | ◎ |
| | | Oil resistance | ◎ | ◎ | ◎ | ◎ | ◎ | ○ | ○ | ◎ | ◎ | ◎ | ◎ | ○ | ◎ | ◎ | ◎ | ◎ | ◎ | ◎ |
| | | Softness | ○ | ○ | ○ | ○ | ○ | ○ | ○ | ○ | ○ | ○ | ○ | ○ | ○ | ○ | ○ | ○ | ○ | ○ |
| | | Tackiness | ○ | ○ | ○ | ○ | ○ | ○ | ○ | ○ | ○ | ○ | ○ | ○ | ○ | ○ | △ | ○ | ○ | ○ |
| | | Odor from volatile organic compound | ○ | ○ | ○ | ○ | ○ | ○ | ○ | ○ | ○ | ○ | ○ | ○ | ○ | ○ | ○ | △ | △ | ○ |
| | | Volatile organic compound (unreacted monomer) level [ppm] | 4 | 2 | 2 | 4 | 3 | 2 | 5 | 7 | 4 | 5 | 4 | 8 | 5 | 4 | 3 | 178 | 169 | 8 |
| | | Alcohol (derived from monomer (A)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | 14 | 12 | ND(<1ppm) |
| | | Methanol (derived from monomer (B)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | 38 | 37 | ND(<1ppm) |
| | | Alcohol (derived from monomer (A)) level [ppm] and methanol (derived from monomer (B)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | 52 | 49 | ND(<1ppm) |
| | | Alcohol (derived from monomer (A)) level [ppm] after long-term storage | 39 | 34 | 25 | 47 | 34 | 30 | 28 | 38 | 37 | 36 | 38 | 39 | 38 | 34 | 41 | 46 | 43 | 46 |
| | | Methanol (derived from monomer (B)) level [ppm] after long-term storage | ND(<1ppm) | ND(<1ppm) | ND(<1ppm) | ND(<1ppm) | ND(<1ppm) | ND(<1ppm) | ND(<1ppm) | 3 | 12 | 44 | 6 | ND(<1ppm) | ND(<1ppm) | ND(<1ppm) | ND(<1ppm) | 50 | 49 | 18 |
| | | Alcohol (derived from monomer (A)) level [ppm] and methanol (derived from monomer (B)) level [ppm] after long-term storage | 39 | 34 | 25 | 47 | 34 | 30 | 28 | 41 | 49 | 80 | 44 | 39 | 38 | 34 | 41 | 96 | 92 | 64 |

[Table 4]

[Table 4]

|  |  |  | Examples |  |  |  |  |  |  |  |  |  |  |  |  |  | Comparative Examples |  |  |  |  |  |
|---|---|---|---|---|---|---|---|---|---|---|---|---|---|---|---|---|---|---|---|---|---|---|---|
|  |  |  | 19 | 20 | 21 | 22 | 23 | 24 | 25 | 26 | 27 | 28 | 29 | 30 | 31 | 32 | 1 | 2 | 3 | 4 | 5 | 6 |
| Evaluation | Liq. Properties | Non-volatile component concentration [%] | 46.2 | 46.3 | 45.5 | 45.6 | 45.5 | 46.8 | 47.2 | 45.9 | 45.8 | 45.8 | 45.9 | 46.1 | 46.2 | 46.0 | 45.9 | 46.0 | 48.7 | 45.8 | 46.2 | 45.8 |
|  |  | Viscosity [mPa.s] | 60 | 110 | 55 | 50 | 50 | 95 | 110 | 50 | 55 | 50 | 55 | 50 | 55 | 50 | 55 | 60 | 350 | 55 | 60 | 55 |
|  |  | pH | 8.0 | 7.9 | 7.8 | 8.1 | 7.9 | 8.0 | 8.1 | 8.0 | 8.1 | 8.0 | 8.0 | 8.1 | 7.9 | 7.9 | 8.1 | 8.0 | 8.0 | 8.0 | 8.1 | 8.0 |
|  |  | Volume av. particle size | 114 | 116 | 109 | 111 | 112 | 125 | 134 | 114 | 113 | 117 | 116 | 118 | 114 | 110 | 114 | 121 | 147 | 115 | 114 | 120 |
|  | Phys properties | Water resistance | ◎ | ◎ | ◎ | ◎ | ◎ | ○ | ○ | ◎ | ◎ | ◎ | ◎ | ○ | ◎ | ◎ | △ | × | × | ○ | ○ | ◎ |
|  |  | Oil resistance | ◎ | ◎ | ◎ | ◎ | ◎ | ◎ | ○ | ○ | ◎ | ◎ | ◎ | ◎ | ◎ | ◎ | ○ | ○ | △ | △ | ◎ | ◎ |
|  |  | Softness | ○ | ○ | ○ | ○ | ○ | ○ | ○ | ○ | ○ | ○ | ○ | ○ | ○ | △ | ○ | × | ○ | ○ | ○ | ○ |
|  |  | Tackiness | ○ | ○ | ○ | ○ | ○ | ○ | △ | ○ | ○ | ○ | ○ | ○ | △ | ◎ | ○ | ○ | × | ○ | ○ | ○ |
|  |  | Odor from volatile organic compound | ○ | ○ | ○ | ○ | ○ | ○ | ○ | ○ | ○ | ○ | ○ | ○ | ○ | ○ | ○ | ○ | ○ | ○ | ○ | ○ |
|  |  | Volatile organic compound (unreacted monomer) level [ppm] | 4 | 5 | 6 | 5 | 10 | 5 | 4 | 5 | 4 | 5 | 6 | 4 | 8 | 5 | 5 | 6 | 4 | 10 | 8 | 10 |
|  |  | Alcohol (derived from monomer (A)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) |
|  |  | Methanol (derived from monomer (B)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) |
|  |  | Alcohol (derived from monomer (A)) level [ppm] and methanol (derived from monomer (B)) level [ppm] immediately after production | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) | ND(<1ppm) |
|  |  | Alcohol (derived from monomer (A)) level [ppm] after long-term storage | 26 | 18 | 50 | 46 | 44 | 26 | 25 | 88 | 74 | 46 | 66 | 54 | 47 | 42 | 48 | 32 | 24 | 106 | 112 | 137 |
|  |  | Methanol (derived from monomer (B)) level [ppm] after long-term storage | 10 | 35 | 20 | 19 | 18 | 11 | 10 | ND(<1ppm) | ND(<1ppm) | ND(<1ppm) | ND(<1ppm) | ND(<1ppm) | ND(<1ppm) | ND(<1ppm) | 119 | ND(<1ppm) | 39 | 107 | 102 | 39 |
|  |  | Alcohol (derived from monomer (A)) level [ppm] and methanol (derived from monomer (B)) level [ppm] after long-term storage | 36 | 53 | 70 | 65 | 62 | 37 | 35 | 88 | 74 | 46 | 66 | 54 | 47 | 42 | 167 | 32 | 63 | 213 | 214 | 176 |

EP 4 609 850 A1

24

Abbreviations in the tables are as follows.

<Monomers>

EMA: ethyl methacrylate derived from petroleum (i.e., non-biomass origin)
BMA: n-butyl methacrylate derived from petroleum (i.e., non-biomass origin)
BA: n-butyl acrylate derived from petroleum (i.e., non-biomass origin)
2EHA: 2-ethylhexyl acrylate derived from petroleum (i.e., non-biomass origin)
LA: biomass-origin lauryl acrylate having a C12 linear-chain alkyl group at an ester end; Biomass acrylate LA (C12 acrylate) (product of Osaka Organic Chemical Industry Ltd.)
MMA: methyl methacrylate derived from petroleum (i.e., non-biomass origin)
MA: methyl acrylate derived from petroleum (i.e., non-biomass origin)
MAA: methacrylic acid derived from petroleum (i.e., non-biomass origin)
AA: acrylic acid derived from petroleum (i.e., non-biomass origin)
St: styrene derived from petroleum (i.e., non-biomass origin)
HEMA: 2-hydroxyethyl methacrylate derived from petroleum (i.e., non-biomass origin)

<Surfactants>

2F-30: Emal 2F-30, sodium laurylsulfate aqueous solution (solid content: 30%) (product of Kao Corporation)
E-120: Emulgen 120, polyoxyethylene lauryl ether (solid content: 100%) (product of Kao Corporation)
E-106: Emulgen 106, polyoxyethylene lauryl ether (solid content: 100%) (product of Kao Corporation)
E-108: Emulgen 108, polyoxyethylene lauryl ether (solid content: 100%) (product of Kao Corporation)
E-109P: Emulgen 109P, polyoxyethylene lauryl ether (solid content: 100%) (product of Kao Corporation)
E-123P: Emulgen 123P, polyoxyethylene lauryl ether (product of Kao Corporation)
E-103: Emulgen 103, polyoxyethylene lauryl ether (solid content: 100%) (product of Kao Corporation)
E-150: Emulgen 150, polyoxyethylene lauryl ether (solid content: 100%) (product of Kao Corporation)

As shown in Tables 3 and 4, the aqueous polymer emulsions of Examples 1 to 32 were found to exhibit suitable water resistance and oil resistance in a well-balanced manner, a low alcohol content, and excellent storage stability, as compared with Comparative Example 1 (the composition of monomer (B) falling outside the scope of the present disclosure); Comparative Example 2 (the composition of monomer (C) falling outside the scope of the present disclosure); Comparative Example 3 (the amount of nonionic surfactant falling outside the scope of the present disclosure); Comparative Examples 4 and 5 (HLB of nonionic surfactant falling outside the scope of the present disclosure); and Comparative Example 6 (omission of heat treatment). In addition, the aqueous polymer emulsions of Examples 1 to 32 were also found to exhibit excellent results in terms of tackiness and low-odor performance.

(2) Production and evaluation of cosmetic compositions

[Example 33]

Production of mascara

To ion-exchanged water (22.3 parts), methylhydroxypropylcellulose (2 parts), talc (10 parts) and the aqueous polymer emulsion obtained in Example 1 (45 parts) were added, and the mixture was uniformly stirred. Subsequently, to the thus-obtained liquid mixture, a color paste containing black iron oxide (15 parts), glycerin (5 parts), and polyoxyethylene sorbitan monooleate (1.5 parts) was added, and the mixture was uniformly mixed through stirring. A perfume (0.1 parts) and an antiseptic (0.1 parts) were added thereto, to thereby yield a black mascara.

[Examples 34 to 48 and Comparative Examples 7 to 11]

Production of mascaras

The procedure of Example 33 was repeated, except that the aqueous polymer emulsion was changed to a corresponding emulsion that shown in Table 5, to thereby yield mascaras of Examples 34 to 48 and Comparative Examples 7 to 11. Notably, the numerical data given in Table 5 without unit refer to those with a unit of "part(s)," and a blank column denotes that no specific item was used (the same applies to Tables 6 to 8).

[Evaluation of mascaras]

Each of the cosmetic compositions (mascaras) of Examples 33 to 48 and Comparative Examples 7 to 11 were evaluated in terms of water resistance, oil resistance, cosmetic effect persistency, and stability over time through the following procedure.

<Water resistance>

A cosmetic composition (mascara) was applied onto a glass plate by means of an applicator so as to have a dry thickness of 50 μm, and air-dried at 23°C and 50%RH for 1 day, to thereby prepare a test piece. The thus-prepared test piece was immersed in distilled water for 1 day, and occurrence of falling and peeling of coating film were evaluated on the basis of the following ratings.

◎: No change was observed in the coating film (good)
○: Slight peeling or falling was observed in the coating film (fair)
Δ: Peeling or falling was observed in about a half portion of the coating film (slightly bad)
X: Peeling or falling was observed in a considerable portion of the coating film (bad)

<Oil resistance>

A test piece prepared through the same method as employed in the aforementioned water resistance test was immersed in oleic acid for 1 day, and occurrence of falling and peeling coating film were evaluated on the basis of the same ratings as employed in the aforementioned water resistance test.

<Cosmetic effect consistency (cosmetic persistency)>

Ten panelists joined to a test of usability for evaluating cosmetic persistency (i.e., cosmetic effect consistency). The ratings of evaluation are as follows.

◎: All 10 panelists evaluated as good.
○: ≥8 of 10 Panelists evaluated as good.
Δ: ≥5 of 10 Panelists evaluated as good.
X: ≤4 of 10 Panelists evaluated as good.

<Stability over time>

A cosmetic composition (40 g) was placed in a glass container (50 mL) and closely sealed in the container with a cap. The container was maintained in a hot air drier at 40°C for 1 month. Thereafter, the state of separation was observed to evaluate stability over time, which was evaluated on the basis of the following ratings.

(Ratings of evaluation)

◎: no separation (very good)
○: slight separation, but disappeared in mixing (good)
Δ: separation, and not disappeared in mixing (slightly bad)
X: considerable separation (bad)

[Table 5]

| | Examples | | | | | | | | | | | | | | | | Comparative Examples | | | | |
|---|---|---|---|---|---|---|---|---|---|---|---|---|---|---|---|---|---|---|---|---|---|
| | 33 | 34 | 35 | 36 | 37 | 38 | 39 | 40 | 41 | 42 | 43 | 44 | 45 | 46 | 47 | 48 | 7 | 8 | 9 | 10 | 11 |
| Aq. emulsion obtained in Ex. 1 | 45 | | | | | | | | | | | | | | | | | | | | |
| Aq. emulsion obtained in Ex. 3 | | 45 | | | | | | | | | | | | | | | | | | | |
| Aq. emulsion obtained in Ex. 5 | | | 45 | | | | | | | | | | | | | | | | | | |
| Aq. emulsion obtained in Ex. 7 | | | | 45 | | | | | | | | | | | | | | | | | |
| Aq. emulsion obtained in Ex. 9 | | | | | 45 | | | | | | | | | | | | | | | | |
| Aq. emulsion obtained in Ex. 11 | | | | | | 45 | | | | | | | | | | | | | | | |
| Aq. emulsion obtained in Ex. 13 | | | | | | | 45 | | | | | | | | | | | | | | |
| Aq. emulsion obtained in Ex. 15 | | | | | | | | 45 | | | | | | | | | | | | | |
| Aq. emulsion obtained in Ex. 17 | | | | | | | | | 45 | | | | | | | | | | | | |
| Aq. emulsion obtained in Ex. 19 | | | | | | | | | | 45 | | | | | | | | | | | |
| Aq. emulsion obtained in Ex. 21 | | | | | | | | | | | 45 | | | | | | | | | | |
| Aq. emulsion obtained in Ex. 23 | | | | | | | | | | | | 45 | | | | | | | | | |
| Aq. emulsion obtained in Ex. 25 | | | | | | | | | | | | | 45 | | | | | | | | |
| Aq. emulsion obtained in Ex. 27 | | | | | | | | | | | | | | 45 | | | | | | | |
| Aq. emulsion obtained in Ex. 29 | | | | | | | | | | | | | | | 45 | | | | | | |
| Aq. emulsion obtained in Ex. 31 | | | | | | | | | | | | | | | | 45 | | | | | |
| Aq. emulsion obtained in Comp. Ex. 1 | | | | | | | | | | | | | | | | | 45 | | | | |
| Aq. emulsion obtained in Comp. Ex. 2 | | | | | | | | | | | | | | | | | | 45 | | | |
| Aq. emulsion obtained in Comp. Ex. 3 | | | | | | | | | | | | | | | | | | | 45 | | |
| Aq. emulsion obtained in Comp. Ex. 4 | | | | | | | | | | | | | | | | | | | | 45 | |
| Aq. emulsion obtained in Comp. Ex. 5 | | | | | | | | | | | | | | | | | | | | | 45 |
| Black iron oxide | 15 | 15 | 15 | 15 | 15 | 15 | 15 | 15 | 15 | 15 | 15 | 15 | 15 | 15 | 15 | 15 | 15 | 15 | 15 | 16 | 15 |
| Talc | 10 | 10 | 10 | 10 | 10 | 10 | 10 | 10 | 10 | 10 | 10 | 10 | 10 | 10 | 10 | 10 | 10 | 10 | 10 | 11 | 10 |
| Methylhydroxypropylcellulose | 2 | 2 | 2 | 2 | 2 | 2 | 2 | 2 | 2 | 2 | 2 | 2 | 2 | 2 | 2 | 2 | 2 | 2 | 2 | 3 | 2 |
| Polyoxyethylene sorbitan monooleate | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 2.5 | 1.5 |
| Glycerin | 5 | 5 | 5 | 5 | 5 | 5 | 5 | 5 | 5 | 5 | 5 | 5 | 5 | 5 | 5 | 5 | 5 | 5 | 5 | 6 | 5 |
| Ion-exchanged water | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 23.3 | 22.3 |
| Perfume | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 1.1 | 0.1 |
| Antiseptic | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 1.1 | 0.1 |
| Evaluation — Water resistance | ◎ | ◎ | ◎ | ○ | ◎ | ○ | ◎ | ○ | ◎ | ◎ | ◎ | ◎ | ○ | ◎ | ○ | ◎ | △ | × | × | ○ | △ |
| Evaluation — Oil resistance | ◎ | ◎ | ◎ | ○ | ◎ | ◎ | ◎ | ◎ | ◎ | ◎ | ◎ | ◎ | ○ | ◎ | ◎ | ◎ | ○ | ○ | △ | △ | ◎ |
| Evaluation — Cosmetic persistency | ◎ | ◎ | ◎ | ○ | ◎ | ◎ | ◎ | ○ | ◎ | ◎ | ◎ | ◎ | ◎ | ◎ | ◎ | ◎ | △ | △ | × | △ | ○ |
| Evaluation — Stability over time | ◎ | ◎ | ◎ | ◎ | ◎ | ◎ | ◎ | ○ | ◎ | ◎ | ◎ | ◎ | ◎ | ◎ | ◎ | ◎ | ◎ | △ | ◎ | ◎ | ◎ |

As shown in Table 5, the cosmetic compositions of Examples 33 to 48 were found to exhibit suitable water resistance, oil resistance, and cosmetic persistency, as compared with Comparative Example 7 (the composition of monomer (B) falling outside the scope of the present disclosure); Comparative Example 8 (the composition of monomer (C) falling outside the scope of the present disclosure); Comparative Example 9 (the amount of nonionic surfactant falling outside the scope of the present disclosure); and Comparative Examples 10 and 11 (HLB of nonionic surfactant falling outside the scope of the present disclosure). In addition, the cosmetic compositions of Examples 33 to 48 were also found to exhibit excellent stability over time.

[Example 49]

Production and evaluation of manicure

A solution prepared by mixing ethylene glycol monoethyl ether (10 parts), acetyl tributyl citrate (3 parts), and ion-exchanged water (7 parts) was gradually added to the aqueous polymer emulsion (80 parts) obtained in Example 2, and stirring was continued to achieve a uniform state, to thereby yield a manicure. The thus-obtained manicure was evaluated in terms of water resistance, oil resistance, cosmetic persistency, and stability over time, through the same evaluation method as employed in the case of mascaras.

[Examples 50 to 64 and Comparative Examples 12 to 16]

Production and evaluation of manicures

The procedure of Example 49 was repeated, except that the emulsion was changed to a corresponding aqueous polymer emulsion shown in Table 6, to thereby yield manicures of Examples 50 to 64 and Comparative Examples 12 to 16. Also, each of the thus-obtained manicures was evaluated in terms of water resistance, oil resistance, cosmetic persistency, and stability over time, through the same evaluation method as employed in the case of mascaras.

[Table 6]

| | | Examples | | | | | | | | | | | | | | | | Comparative Examples | | | | |
|---|---|---|---|---|---|---|---|---|---|---|---|---|---|---|---|---|---|---|---|---|---|---|---|
| | | 49 | 50 | 51 | 52 | 53 | 54 | 55 | 56 | 57 | 58 | 59 | 60 | 61 | 62 | 63 | 64 | 12 | 13 | 14 | 15 | 16 |
| Aq. emulsion obtained in Ex. 2 | | 80 | | | | | | | | | | | | | | | | | | | | |
| Aq. emulsion obtained in Ex. 4 | | | 80 | | | | | | | | | | | | | | | | | | | |
| Aq. emulsion obtained in Ex. 6 | | | | 80 | | | | | | | | | | | | | | | | | | |
| Aq. emulsion obtained in Ex. 8 | | | | | 80 | | | | | | | | | | | | | | | | | |
| Aq. emulsion obtained in Ex. 10 | | | | | | 80 | | | | | | | | | | | | | | | | |
| Aq. emulsion obtained in Ex. 12 | | | | | | | 80 | | | | | | | | | | | | | | | |
| Aq. emulsion obtained in Ex. 14 | | | | | | | | 80 | | | | | | | | | | | | | | |
| Aq. emulsion obtained in Ex. 16 | | | | | | | | | 80 | | | | | | | | | | | | | |
| Aq. emulsion obtained in Ex. 18 | | | | | | | | | | 80 | | | | | | | | | | | | |
| Aq. emulsion obtained in Ex. 20 | | | | | | | | | | | 80 | | | | | | | | | | | |
| Aq. emulsion obtained in Ex. 22 | | | | | | | | | | | | 80 | | | | | | | | | | |
| Aq. emulsion obtained in Ex. 24 | | | | | | | | | | | | | 80 | | | | | | | | | |
| Aq. emulsion obtained in Ex. 26 | | | | | | | | | | | | | | 80 | | | | | | | | |
| Aq. emulsion obtained in Ex. 28 | | | | | | | | | | | | | | | 80 | | | | | | | |
| Aq. emulsion obtained in Ex. 30 | | | | | | | | | | | | | | | | 80 | | | | | | |
| Aq. emulsion obtained in Ex. 32 | | | | | | | | | | | | | | | | | 80 | | | | | |
| Aq. emulsion obtained in Comp. Ex. 1 | | | | | | | | | | | | | | | | | | 80 | | | | |
| Aq. emulsion obtained in Comp. Ex. 2 | | | | | | | | | | | | | | | | | | | 80 | | | |
| Aq. emulsion obtained in Comp. Ex. 3 | | | | | | | | | | | | | | | | | | | | 80 | | |
| Aq. emulsion obtained in Comp. Ex. 4 | | | | | | | | | | | | | | | | | | | | | 80 | |
| Aq. emulsion obtained in Comp. Ex. 5 | | | | | | | | | | | | | | | | | | | | | | 80 |
| Ethylene glycol monoethyl ether | | 10 | 10 | 10 | 10 | 10 | 10 | 10 | 10 | 10 | 10 | 10 | 10 | 10 | 10 | 10 | 10 | 10 | 10 | 10 | 10 | 10 |
| Acetyl tributyl citrate | | 3 | 3 | 3 | 3 | 3 | 3 | 3 | 3 | 3 | 3 | 3 | 3 | 3 | 3 | 3 | 3 | 3 | 3 | 3 | 3 | 3 |
| Ion-exchanged water | | 7 | 7 | 7 | 7 | 7 | 7 | 7 | 7 | 7 | 7 | 7 | 7 | 7 | 7 | 7 | 7 | 7 | 7 | 7 | 7 | 7 |
| Evaluation | Water resistance | ◎ | ◎ | ◎ | ◎ | ◎ | ◎ | ○ | ◎ | ◎ | ◎ | ◎ | ○ | ◎ | ◎ | ○ | ◎ | ○ | × | × | △ | △ |
| | Oil resistance | ◎ | ◎ | ○ | ◎ | ◎ | ○ | ◎ | ◎ | ◎ | ◎ | ◎ | ◎ | ○ | ◎ | ◎ | ◎ | △ | △ | △ | △ | ◎ |
| | Cosmetic persistency | ◎ | ◎ | ○ | ◎ | ◎ | ○ | ○ | ◎ | ◎ | ◎ | ◎ | ◎ | ○ | ◎ | ◎ | ◎ | △ | △ | × | △ | ○ |
| | Stability over time | ◎ | ◎ | ◎ | ◎ | ◎ | ○ | ○ | ◎ | ◎ | ◎ | ◎ | ◎ | ◎ | ◎ | ◎ | ◎ | ◎ | △ | ◎ | ◎ | ◎ |

As shown in Table 6, the cosmetic compositions of Examples 49 to 64 were found to exhibit more excellent water resistance, oil resistance, and cosmetic persistency, as compared with Comparative Example 12 (the composition of monomer (B) falling outside the scope of the present disclosure); Comparative Example 13 (the composition of monomer (C) falling outside the scope of the present disclosure); Comparative Example 14 (the amount of nonionic surfactant falling outside the scope of present disclosure); and Comparative Examples 15 and 16 (the HBL of nonionic surfactant falling outside the scope of present disclosure). In addition, the cosmetic compositions of Examples 49 to 64 were also found to exhibit excellent results in terms of stability over time.

[Example 65]

Production and evaluation of eyeliner

Polyoxyethylene (20) sorbitan monooleate (1 part), carboxymethylcellulose (0.15 parts), and iron oxide (14 parts) were added to ion-exchanged water (33.9 parts), and the mixture was uniformly mixed through stirring. Subsequently, glycerin (5 parts) and acetyl tributyl citrate (1 part) were added thereto, and the aqueous polymer emulsion obtained in Example 1 (45 parts) was gradually added. The formulation was stirred to achieve a uniform state, to thereby yield an eyeliner. The thus-obtained eyeliner was evaluated in terms of water resistance, oil resistance, cosmetic persistency, and stability over time, through the same evaluation method as employed in the case of mascaras.

[Examples 66 to 83 and Comparative Examples 17 to 21]

Production and evaluation of eyeliners

The procedure of Example 65 was repeated, except that the formulation was changed to a corresponding one shown in Table 7, to thereby yield eyeliners of Examples 66 to 80 and Comparative Examples 17 to 21. Also, each of the thus-obtained eyeliners was evaluated in terms of water resistance, oil resistance, cosmetic persistency, and stability over time, through the same evaluation method as employed in the case of mascaras.

[Table 7]

| | Examples | | | | | | | | | | | | | | | | Compalative Examples | | | | |
|---|---|---|---|---|---|---|---|---|---|---|---|---|---|---|---|---|---|---|---|---|---|
| | 65 | 66 | 67 | 68 | 69 | 70 | 71 | 72 | 73 | 74 | 75 | 76 | 77 | 78 | 79 | 80 | 17 | 18 | 19 | 20 | 21 |
| Aq. emulsion obtained in Ex. 1 | 45 | | | | | | | | | | | | | | | | | | | | |
| Aq. emulsion obtained in Ex. 3 | | 45 | | | | | | | | | | | | | | | | | | | |
| Aq. emulsion obtained in Ex. 5 | | | 45 | | | | | | | | | | | | | | | | | | |
| Aq. emulsion obtained in Ex. 7 | | | | 45 | | | | | | | | | | | | | | | | | |
| Aq. emulsion obtained in Ex. 9 | | | | | 45 | | | | | | | | | | | | | | | | |
| Aq. emulsion obtained in Ex. 11 | | | | | | 45 | | | | | | | | | | | | | | | |
| Aq. emulsion obtained in Ex. 13 | | | | | | | 45 | | | | | | | | | | | | | | |
| Aq. emulsion obtained in Ex. 15 | | | | | | | | 45 | | | | | | | | | | | | | |
| Aq. emulsion obtained in Ex. 17 | | | | | | | | | 45 | | | | | | | | | | | | |
| Aq. emulsion obtained in Ex. 19 | | | | | | | | | | 45 | | | | | | | | | | | |
| Aq. emulsion obtained in Ex. 21 | | | | | | | | | | | 45 | | | | | | | | | | |
| Aq. emulsion obtained in Ex. 23 | | | | | | | | | | | | 45 | | | | | | | | | |
| Aq. emulsion obtained in Ex. 25 | | | | | | | | | | | | | 45 | | | | | | | | |
| Aq. emulsion obtained in Ex. 27 | | | | | | | | | | | | | | 45 | | | | | | | |
| Aq. emulsion obtained in Ex. 29 | | | | | | | | | | | | | | | 45 | | | | | | |
| Aq. emulsion obtained in Ex. 31 | | | | | | | | | | | | | | | | 45 | | | | | |
| Aq. emulsion obtained in Comp. Ex. 1 | | | | | | | | | | | | | | | | | 45 | | | | |
| Aq. emulsion obtained in Comp. Ex. 2 | | | | | | | | | | | | | | | | | | 45 | | | |
| Aq. emulsion obtained in Comp. Ex. 3 | | | | | | | | | | | | | | | | | | | 45 | | |
| Aq. emulsion obtained in Comp. Ex. 4 | | | | | | | | | | | | | | | | | | | | 45 | |
| Aq. emulsion obtained in Comp. Ex. 5 | | | | | | | | | | | | | | | | | | | | | 45 |
| Iron oxide | 14 | 14 | 14 | 14 | 14 | 14 | 14 | 14 | 14 | 14 | 14 | 14 | 14 | 14 | 14 | 14 | 14 | 14 | 14 | 14 | 14 |
| Glycerin | 5 | 5 | 5 | 5 | 5 | 5 | 5 | 5 | 5 | 5 | 5 | 5 | 5 | 5 | 5 | 5 | 5 | 5 | 5 | 5 | 5 |
| Polyoxyethylene(20) sorbitan monooleate ester | 1 | 1 | 1 | 1 | 1 | 1 | 1 | 1 | 1 | 1 | 1 | 1 | 1 | 1 | 1 | 1 | 1 | 1 | 1 | 1 | 1 |
| Carboxymethylcellulose | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 |
| Acetyl tributyl citrate | 1 | 1 | 1 | 1 | 1 | 1 | 1 | 1 | 1 | 1 | 1 | 1 | 1 | 1 | 1 | 1 | 1 | 1 | 1 | 1 | 1 |
| Ion-exchanged water | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 |
| Evaluation — Water resistance | ◎ | ◎ | ◎ | ○ | ◎ | ◎ | ◎ | ○ | ◎ | ◎ | ◎ | ◎ | ○ | ◎ | ○ | ◎ | △ | × | × | ○ | △ |
| Evaluation — Oil resistance | ◎ | ◎ | ◎ | ○ | ◎ | ◎ | ◎ | ◎ | ◎ | ◎ | ◎ | ◎ | ◎ | ◎ | ◎ | ◎ | △ | ○ | △ | △ | ○ |
| Evaluation — Cosmetic persistency | ◎ | ◎ | ◎ | ○ | ◎ | ◎ | ○ | ◎ | ◎ | ◎ | ◎ | ◎ | ◎ | ◎ | ◎ | ◎ | △ | △ | × | △ | △ |
| Evaluation — Stability over time | ◎ | ◎ | ◎ | ◎ | ◎ | ◎ | ◎ | ○ | ◎ | ◎ | ◎ | ◎ | ◎ | ◎ | ◎ | ◎ | ◎ | △ | ◎ | ◎ | ◎ |

As shown in Table 7, the cosmetic compositions of Examples 65 to 80 were found to exhibit more excellent water resistance, oil resistance, and cosmetic persistency, as compared with Comparative Example 17 (the composition of monomer (B) falling outside the scope of the present disclosure); Comparative Example 18 (the composition of monomer (C) falling outside the scope of the present disclosure); Comparative Example 19 (the amount of nonionic surfactant falling outside the scope of present disclosure); and Comparative Examples 20 and 21 (the HBL of nonionic surfactant falling outside the scope of present disclosure). In addition, the cosmetic compositions of Examples 65 to 80 were also found to exhibit excellent results in terms of stability over time.

[Example 81]

Production and evaluation of foundation

The aqueous polymer emulsion obtained in Example 2 (5 parts), bentonite (0.5 parts), polyoxyethylene sorbitan monostearate (1 part), propylene glycol (10 parts), an antiseptic (0.1 parts), and ion-exchanged water (52.4 parts) were mixed, and the ingredients were dispersed to a uniform state at 70°C by means of a homo mixer. The pH of the mixture was adjusted to 7.0 with triethanolamine. Subsequently, talc (3 parts), titanium dioxide (5 parts), red iron oxide (0.5 parts), and iron oxide (1 part) were sufficiently mixed, and the mixture was added to the above-prepared dispersion. Stearic acid (2.5 parts), isohexadecyl alcohol (7 parts), glycerin monostearate (2 parts), liquid lanolin (2 parts), and liquid paraffin (8 parts) were mixed, and the ingredients were dissolved at 80°C, to thereby form an oil phase. The oil phase was added to the above-prepared aqueous phase, and the mixture was processed by means of a homo mixer at 70°C for emulsification. Thereafter, the emulsion was cooled to ambient temperature, to thereby yield a foundation. The thus-obtained foundation was evaluated in terms of water resistance, oil resistance, cosmetic persistency, and stability over time, through the same evaluation method as employed in the case of mascaras.

[Examples 82 to 96 and Comparative Examples 22 to 26]

Production and evaluation of foundations

The procedure of Example 81 was repeated, except that the formulation was changed to a corresponding one shown in Table 8, to thereby yield foundations of Examples 82 to 96 and Comparative Examples 22 to 26. Also, each of the thus-obtained foundations was evaluated in terms of water resistance, oil resistance, cosmetic persistency, and stability over time, through the same evaluation method as employed in the case of mascaras.

[Table 8]

| | Examples | | | | | | | | | | | | | | | | Comparative Examples | | | | |
| --- | 81 | 82 | 83 | 84 | 85 | 86 | 87 | 88 | 89 | 90 | 91 | 92 | 93 | 94 | 95 | 96 | 22 | 23 | 24 | 25 | 26 |
| --- | --- | --- | --- | --- | --- | --- | --- | --- | --- | --- | --- | --- | --- | --- | --- | --- | --- | --- | --- | --- | --- |
| Aq. emulsion obtained in Ex. 2 | 5 | | | | | | | | | | | | | | | | | | | | |
| Aq. emulsion obtained in Ex. 4 | | 5 | | | | | | | | | | | | | | | | | | | |
| Aq. emulsion obtained in Ex. 6 | | | 5 | | | | | | | | | | | | | | | | | | |
| Aq. emulsion obtained in Ex. 8 | | | | 5 | | | | | | | | | | | | | | | | | |
| Aq. emulsion obtained in Ex. 10 | | | | | 5 | | | | | | | | | | | | | | | | |
| Aq. emulsion obtained in Ex. 12 | | | | | | 5 | | | | | | | | | | | | | | | |
| Aq. emulsion obtained in Ex. 14 | | | | | | | 5 | | | | | | | | | | | | | | |
| Aq. emulsion obtained in Ex. 16 | | | | | | | | 5 | | | | | | | | | | | | | |
| Aq. emulsion obtained in Ex. 18 | | | | | | | | | 5 | | | | | | | | | | | | |
| Aq. emulsion obtained in Ex. 20 | | | | | | | | | | 5 | | | | | | | | | | | |
| Aq. emulsion obtained in Ex. 22 | | | | | | | | | | | 5 | | | | | | | | | | |
| Aq. emulsion obtained in Ex. 24 | | | | | | | | | | | | 5 | | | | | | | | | |
| Aq. emulsion obtained in Ex. 26 | | | | | | | | | | | | | 5 | | | | | | | | |
| Aq. emulsion obtained in Ex. 28 | | | | | | | | | | | | | | 5 | | | | | | | |
| Aq. emulsion obtained in Ex. 30 | | | | | | | | | | | | | | | 5 | | | | | | |
| Aq. emulsion obtained in Ex. 32 | | | | | | | | | | | | | | | | 5 | | | | | |
| Aq. emulsion obtained in Comp. Ex. 1 | | | | | | | | | | | | | | | | | 5 | | | | |
| Aq. emulsion obtained in Comp. Ex. 2 | | | | | | | | | | | | | | | | | | 5 | | | |
| Aq. emulsion obtained in Comp. Ex. 3 | | | | | | | | | | | | | | | | | | | 5 | | |
| Aq. emulsion obtained in Comp. Ex. 4 | | | | | | | | | | | | | | | | | | | | 5 | |
| Aq. emulsion obtained in Comp. Ex. 5 | | | | | | | | | | | | | | | | | | | | | 5 |
| Bentonite | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Polyoxyethylene sorbitan monostearate | 1 | 1 | 1 | 1 | 1 | 1 | 1 | 1 | 1 | 1 | 1 | 1 | 1 | 1 | 1 | 1 | 1 | 1 | 1 | 1 | 1 |
| Propylene glycol | 10 | 10 | 10 | 10 | 10 | 10 | 10 | 10 | 10 | 10 | 10 | 10 | 10 | 10 | 10 | 10 | 10 | 10 | 10 | 10 | 10 |
| Triethanolamine (to pH 7) | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount |
| Antiseptic | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Ion-exchanged water | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 |
| Talc | 3 | 3 | 3 | 3 | 3 | 3 | 3 | 3 | 3 | 3 | 3 | 3 | 3 | 3 | 3 | 3 | 3 | 3 | 3 | 3 | 3 |
| Titanium dioxide | 5 | 5 | 5 | 5 | 5 | 5 | 5 | 5 | 5 | 5 | 5 | 5 | 5 | 5 | 5 | 5 | 5 | 5 | 5 | 5 | 5 |
| Red iron oxide | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Iron oxide | 1 | 1 | 1 | 1 | 1 | 1 | 1 | 1 | 1 | 1 | 1 | 1 | 1 | 1 | 1 | 1 | 1 | 1 | 1 | 1 | 1 |
| Stearic acid | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 |
| Isohexadecyl alcohol | 7 | 7 | 7 | 7 | 7 | 7 | 7 | 7 | 7 | 7 | 7 | 7 | 7 | 7 | 7 | 7 | 7 | 7 | 7 | 7 | 7 |
| Glycerin monostearate | 2 | 2 | 2 | 2 | 2 | 2 | 2 | 2 | 2 | 2 | 2 | 2 | 2 | 2 | 2 | 2 | 2 | 2 | 2 | 2 | 2 |
| Liq. Lanoline | 2 | 2 | 2 | 2 | 2 | 2 | 2 | 2 | 2 | 2 | 2 | 2 | 2 | 2 | 2 | 2 | 2 | 2 | 2 | 2 | 2 |
| Liq. Paraffin | 8 | 8 | 8 | 8 | 8 | 8 | 8 | 8 | 8 | 8 | 8 | 8 | 8 | 8 | 8 | 8 | 8 | 8 | 8 | 8 | 8 |
| Evaluation Water resistance | ◎ | ◎ | ◎ | ◎ | ◎ | ◎ | ○ | ◎ | ◎ | ◎ | ◎ | ○ | ○ | ◎ | ○ | ◎ | △ | × | × | △ | △ |
| Evaluation Oil resistance | ◎ | ◎ | ○ | ◎ | ◎ | ○ | ◎ | ◎ | ◎ | ◎ | ◎ | ◎ | ○ | ◎ | ◎ | ◎ | △ | △ | △ | △ | ○ |
| Evaluation Cosmetic persistency | ◎ | ◎ | ○ | ◎ | ◎ | ○ | ◎ | ◎ | ◎ | ◎ | ◎ | ◎ | ○ | ◎ | ◎ | ◎ | △ | △ | × | △ | △ |
| Evaluation Stability over time | ◎ | ◎ | ◎ | ◎ | ◎ | ○ | ○ | ◎ | ◎ | ◎ | ◎ | ◎ | ◎ | ◎ | ◎ | ◎ | ◎ | △ | ◎ | ◎ | ◎ |

As shown in Table 8, the cosmetic compositions of Examples 81 to 96 were found to exhibit more excellent water resistance, oil resistance, and cosmetic persistency, as compared with Comparative Example 22 (the composition of monomer (B) falling outside the scope of the present disclosure); Comparative Example 23 (the composition of monomer (C) falling outside the scope of the present disclosure); Comparative Example 24 (the amount of nonionic surfactant falling outside the scope of present disclosure); and Comparative Examples 25 and 26 (the HBL of nonionic surfactant falling outside the scope of present disclosure). In addition, the cosmetic compositions of Examples 81 to 96 were also found to exhibit excellent results in terms of stability over time.

The present invention should not be limited to the aforementioned embodiments and encompasses various modifications and modification within a scope of equivalent, so long as they do not deviate from the gist of the present invention. Thus, those skilled in the art should understand that, in reference to the aforementioned teachings, various combinations and modes, and further, those including only one element, one or more elements, or one or less element fall within the scope or concept of the present invention.

**Claims**

1. An aqueous polymer emulsion for cosmetics, the emulsion comprising resin particles, wherein

   the resin particles comprise a structural unit derived from a monomer (A), a structural unit derived from a monomer (C), and an optional structural unit derived from a monomer (B), wherein
   the monomer (A) is an alkyl (meth)acrylate having a C2 to C12 alkyl group;
   the monomer (B) is an unsaturated monomer having a methyl ester group; and
   the monomer (C) is at least one monomer selected from the group consisting of an unsaturated carboxylic acid and an unsaturated carboxylic anhydride;
   the amount of the structural unit derived from the monomer (A) is 70 to 99.9 mass%, the amount of the structural unit derived from the monomer (B) is 0 to 10 mass%, and the amount of the structural unit derived from the monomer (C) is 0.1 to 10 mass%, with respect to the total amount of the structural units derived from the monomers forming the resin particles as 100 mass%; and
   the emulsion comprises a nonionic surfactant having an HLB of 10.0 to 17.0 in an amount of 0.1 to 10 parts by mass, with respect to the total amount of the monomers forming the resin particles as 100 parts by mass; and
   the emulsion has an alcohol level of 150 ppm or lower, the alcohol being derived from an alkyl group comprised in the monomers forming the resin particles.

2. The aqueous polymer emulsion for cosmetics according to claim 1, wherein the polymer forming the resin particles has a glass transition temperature of -20°C to 20°C.

3. The aqueous polymer emulsion for cosmetics according to claim 1, wherein the monomer (C) is methacrylic acid.

4. The aqueous polymer emulsion for cosmetics as according to claim 1, wherein the monomer (B) is methyl (meth)acrylate.

5. A method for producing an aqueous polymer emulsion for cosmetics according to any one of claims 1 to 4, the method comprising

   a step of producing a polymer emulsion by conducting emulsion polymerization, under such conditions that the amount of the structural unit derived from the monomer (A) is 70 to 99.9 mass%, the amount of the structural unit derived from the monomer (B) is 0 to 10 mass%, and the amount of the structural unit derived from the monomer (C) is 0.1 to 10 mass%, with respect to the total amount of the monomers used in the polymerization as 100 mass%; and a nonionic surfactant having an HLB of 10.0 to 17.0 is present in an amount of 0.1 to 10 parts by mass, with respect to the total amount of the monomers used in the polymerization as 100 parts by mass;
   a step including adding a basic compound to the polymer emulsion, to thereby neutralize the emulsion to a pH of 7.0 to 10.0, and heating at 50 to 90°C; and
   a step of removing a volatile organic compound in the aqueous polymer emulsion by supplying pressurized steam to a processing container that can provide the polymer emulsion after being heated with a reduced pressure, while the polymer emulsion is maintained in the processing container at a pressure controlled to 12 kPa to 57 kPa and in an internal state of the processing container as a water boiling condition.

6. A cosmetic comprising an aqueous polymer emulsion for cosmetics according to any one of claims 1 to 4.

## INTERNATIONAL SEARCH REPORT

| International application No. |
| --- |
| **PCT/JP2023/038016** |

**A.    CLASSIFICATION OF SUBJECT MATTER**

*A61K 8/81*(2006.01)i; *A61K 8/06*(2006.01)i; *A61Q 1/00*(2006.01)i
FI:    A61K8/81; A61K8/06; A61Q1/00

According to International Patent Classification (IPC) or to both national classification and IPC

**B.    FIELDS SEARCHED**

Minimum documentation searched (classification system followed by classification symbols)

A61K8/81; A61K8/06; A61Q1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2021/095604 A1 (TOAGOSEI CO., LTD.) 20 May 2021 (2021-05-20) claims, paragraphs [0010], [0012], [0041], [0071], [0090], [0091] | 1-6 |
| X | WO 2021/241091 A1 (NIKKO CHEMICALS) 02 December 2021 (2021-12-02) claims, paragraphs [0006], [0007], [0021], [0022], [0035], [0062], [0063], [0086], [0087] | 1-6 |
| A | JP 1-203313 A (SHISEIDO CO., LTD.) 16 August 1989 (1989-08-16) entire text | 1-6 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 December 2023** | **26 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/038016**

| Patent document cited in search report | | | Publication date (day/month/year) | Patent family member(s) | Publication date (day/month/year) |
|---|---|---|---|---|---|
| WO | 2021/095604 | A1 | 20 May 2021 | (Family: none) | |
| WO | 2021/241091 | A1 | 02 December 2021 | (Family: none) | |
| JP | 1-203313 | A | 16 August 1989 | (Family: none) | |

Form PCT/ISA/210 (patent family annex) (January 2015)

**REFERENCES CITED IN THE DESCRIPTION**

**Patent documents cited in the description**

- JP 2022172406 A

- JP 2000355532 A